# EUROPEAN PATENT SPECIFICATION

(11) **EP 2 432 430 B1**
(45) Date of publication and mention of the grant of the patent: **16.09.2015**
(21) Application number: 10711812.7
(22) Date of filing: 31.03.2010
(51) Int. Cl.: A61F 2/64

(54) **PROSTHETIC KNEE**
KNIEPROTHESE
PROTHÈSE DE GENOU

(43) Date of publication of application: 28.03.2012
(73) Proprietor: LIMBS International Inc., El Paso TX 79901 (US)
(72) Inventor: GONZALEZ, Roger V., Longview Texas 75605 (US); AYERS, Stephen Ronald, Longview Texas 75601 (US); MINELGA, Eric, Stanwood Washington 98292 (US); DOWNING, Wes Lee, Kilgore Texas 75662 (US); FISHER, John Baxter, Lindale Texas 75771 (US)
(74) Representative: Conroy, John
(86) International application number: PCT/US2010/029448
(87) International publication number: WO 2011/123119

(56) References cited:
- EP-A2- 1 849 439
- WO-A1-2009/145753
- DE-C- 417 623
- DE-C- 808 743
- GB-A- 1 418 496
- US-A- 4 178 642
- US-A1- 2005 154 473
- US-A1- 2008 071 388

## Description

### BACKGROUND

Transfemoral amputees often wear prostheses on their residual limb for functional, cosmetic, and other purposes. Many lower-limb prostheses include a prosthetic knee joint that can emulate aspects of a human knee, allowing the amputee to stand, walk, sit, run, and perform other activities while wearing the prosthetic limb. Some prosthetic knee joints utilize a single-axis joint mechanism in which the bending motion of the knee defines a fixed center of rotation throughout the bending motion of the knee. Other prosthetic knee joints utilize a multi-axis joint system in which the bending motion of the knee defines a non-stationary center of rotation. In a multi-axis knee joint, the instantaneous center of rotation translates with respect to the knee during the bending motion of the knee. Such polycentric joints provide advantages such as added stability and improved toe clearance during the swinging motion of the prosthesis.

Many prosthetic knees are built by manufacturing processes that require expensive materials, high-tech manufacturing facilities, and highly skilled technicians and engineers. As a result, such prosthetic devices can be quite expensive, impractical for certain types of use, and unavailable for many transfemoral amputees. For example, in various regions of the world, financial constraints, import regulations, lack of access to materials, lack of access to trained personnel, and other considerations can affect the availability of certain prosthetic devices. Consequently, many transfemoral amputees have been relegated to primitive prosthetic devices. As another example, the cost of replacing or repairing prosthetic devices discourages certain types of use that could damage the prosthetic device. Consequently, the cost associated with replacing or repairing a prosthetic device limits the activities of many transfemoral amputees.

EP 1849439 (A2) alleges to describe a knee prosthetic joint that has four axel bolts. Two opposite joint limbs running diagonally are connected with a prosthetic shaft on one side and connected with a prosthetic foot on the other side. The other two joint limbs are rotated with each other as longitudinal joint limbs in the standing position and the deflecting position. The axel bolt of the joint limb rotates and determines eccentric element connected with the prosthetic foot, by whose rotation the distance between both the axel bolt change at the joint limb.

DE 808743 (C) alleges to describe an artificial knee joint that consists of two pairs of articulated rods on either side of the thigh joint part and hinged to the lower leg joint part are such that a respective hinge for the end pairs of front and rear articulated rods on the upper and also provided on the lower leg, said rear pair of articulated rods front considerably shorter than that.

WO 2009145753 (A1) alleges to describe a prosthetic limb that has an outer surface that is a mirror image of an intact limb or a generic limb design. The intact limb is scanned and the surface data is manipulated to create a virtual mirror image. If generic data is used, the intact leg can be measured and the generic surface can be adjusted so the prosthetic limb appears similar to the intact limb. The end of the amputated limb is also measured to obtain socket data. A knee and foot are incorporated to form a virtual prosthetic limb represented by design data. The design data for the virtual prosthetic limb is forwarded to a rapid prototyping machine that fabricates the entire leg simultaneously. Once completed, the prosthetic limb is shipped to the patient.

US 4178642 (A) alleges to describe an artificial leg of the kind in which the shin portion and the knee portion are shaped from a block of wood or similar solid material, has the knee and shin portions connected by pairs of front and rear links, the effective length of the front links being less than that of the rear links and the connections between the links and the knee and shin portions being positioned so as to form an articulation of which the instantaneous center of rotation lies upon an ascending curve for a predetermined angle of relative rotation between the two portions of the leg from the fully extended position. The resultant joint allegedly provides a stable weight support until it has been flexed through the predetermined angle and is thereafter capable of flexure through more than a right angle.

DE 417623 alleges to describe an artificial knee joint in which two pairs of articulated rods are provided on either side of the thigh part and the lower leg part are articulated such that a bolt-joint for the end pairs of front and rear articulated rods on top and also on the lower leg are present.

US 2008/0071388 alleges to describe a prosthetic knee joint that includes a knee seat having a slant screw hole, a movable member disposed pivotally in a bottom portion of the knee seat and having a lower mounting portion and an upper pushing portion, two shafts disposed respectively within shaft holes in the lower mounting portion, two C-shaped elastic sleeves sleeved respectively on the shafts and abutting against the upper pushing portion, a linkage connected to the shafts, a weight-buffering device having a rubber cushion disposed directly under the slant screw hole, a buffer adjustment device having a buffer cushion that protrudes outwardly of the movable member to abut against a projection of the knee seat and that is removable from the projection, and a restoring device operable so as to move a free end of the upper pushing portion relative to the lower mounting portion.

US 2005/0154473 alleges to describe a prosthetic knee mechanism that improves stability and flexion of an individual during use. The prosthetic knee mechanism has a braking mechanism to allegedly increase stance phase stability in a polycentric knee. The combination of the braking mechanism with a polycentric knee allegedly increase stance stability and reduces frictional forces on the prosthetic knee components. The prosthetic knee mechanism uses a cam mechanism to control the flexion and extension of the knee. The cam mechanism allegedly also controls the speed at which the foot and shank swing. The prosthetic knee uses a collapsible posterior linkage which allegedly allows the knee to go into a small degree of flexion at heel strike while maintaining overall knee stability.

GB1418496 (A) alleges to describe an artificial knee joint that comprises a resilient plastics molding having a curved anticlastic lower face and a flat upper face having a polygonal opening giving access to an interior space bounded in part by interior surfaces 9, 10 adapted to engage with said artificial knee joint. The surfaces allegedly provide bosses for the pivots of a forward pair of links and bosses for the pivots of a rearward pair of links, slots in the base and rear of the fairing permitting pivoting movement of said links. A strap fixed to the rear upper face of the fairing allegedly provides means of attachment of the fairing to the artificial leg.

### SUMMARY

In a general aspect, a prosthetic knee includes an upper member, a lower member, and linkages connecting the upper member and the lower member. In another general aspect, a drilling jig is used to manufacture component parts of a prosthetic knee. In a general aspect, a graphical manufacturing guide provides graphical instructions for manufacturing a polycentric prosthetic knee.

In some aspects, a prosthetic knee includes an upper member, a lower member, and links connecting axes in the upper member and the lower member. The upper member, the lower member, and the links may be made of non-metal material, including plastics, woods, also provided on the lower leg, said rear pair of articulated rods front considerably shorter than that.

WO 2009145753 (A1) alleges to describe a prosthetic limb that has an outer surface that is a mirror image of an intact limb or a generic limb design. The intact limb is scanned and the surface data is manipulated to create a virtual mirror image. If generic data is used, the intact leg can be measured and the generic surface can be adjusted so the prosthetic limb appears similar to the intact limb. The end of the amputated limb is also measured to obtain socket data. A knee and foot are incorporated to form a virtual prosthetic limb represented by design data. The design data for the virtual prosthetic limb is forwarded to a rapid prototyping machine that fabricates the entire leg simultaneously. Once completed, the prosthetic limb is shipped to the patient.

US 4178642 (A) alleges to describe an artificial leg of the kind in which the shin portion and the knee portion are shaped from a block of wood or similar solid material, has the knee and shin portions connected by pairs of front and rear links, the effective length of the front links being less than that of the rear links and the connections between the links and the knee and shin portions being positioned so as to form an articulation of which the instantaneous center of rotation lies upon an ascending curve for a predetermined angle of relative rotation between the two portions of the leg from the fully extended position. The resultant joint allegedly provides a stable weight support until it has been flexed through the predetermined angle and is thereafter capable of flexure through more than a right angle.

DE 417623 alleges to describe an artificial knee joint in which two pairs of articulated rods are provided on either side of the thigh part and the lower leg part are articulated such that a bolt-joint for the end pairs of front and rear articulated rods on top and also on the lower leg are present.

US 2008/0071388 alleges to describe a prosthetic knee joint that includes a knee seat having a slant screw hole, a movable member disposed pivotally in a bottom portion of the knee seat and having a lower mounting portion and an upper pushing portion, two shafts disposed respectively within shaft holes in the lower mounting portion, two C-shaped elastic sleeves sleeved respectively on the shafts and abutting against the upper pushing portion, a linkage connected to the shafts, a weight-buffering device having a rubber cushion disposed directly under the slant screw hole, a buffer adjustment device having a buffer cushion that protrudes outwardly of the movable member to abut against a projection of the knee seat and that is removable from the projection, and a restoring device operable so as to move a free end of the upper pushing portion relative to the lower mounting portion.

US 2005/0154473 alleges to describe a prosthetic knee mechanism that improves stability and flexion of an individual during use. The prosthetic knee mechanism has a braking mechanism to allegedly increase stance phase stability in a polycentric knee. The combination of the braking mechanism with a polycentric knee allegedly increase stance stability and reduces frictional forces on the prosthetic knee components. The prosthetic knee mechanism uses a cam mechanism to control the flexion and extension of the knee. The cam mechanism allegedly also controls the speed at which the foot and shank swing. The prosthetic knee uses a collapsible posterior linkage which allegedly allows the knee to go into a small degree of flexion at heel strike while maintaining overall knee stability.

GB1418496 (A) alleges to describe an artificial knee joint that comprises a resilient plastics molding having a curved anticlastic lower face and a flat upper face having a polygonal opening giving access to an interior space bounded in part by interior surfaces 9, 10 adapted to engage with said artificial knee joint. The surfaces allegedly provide bosses for the pivots of a forward pair of links and bosses for the pivots of a rearward pair of links, slots in the base and rear of the fairing permitting pivoting movement of said links. A strap fixed to the rear upper face of the fairing allegedly provides means of attachment of the fairing to the artificial leg.

### SUMMARY

The invention relates to a polycentric prosthetic knee as defined in claim 1 and to a method of manufacturing a polycentric prosthetic knee as defined in claim 11.

Implementations of preferred embodiments may include one or more of the following features. The first link and the third link may be anterior axes, and the second link may be a posterior axis. The polycentric prosthetic knee may include an extension assist mechanism that biases the prosthetic knee toward an extended position. The extension assist mechanism may include a rubber band secured to the plastic upper member and to the plastic lower member. The rubber band biases the prosthetic knee toward an extended position. The extension assist mechanism may alternatively include a coil spring having a first end secured to the plastic upper member and a second end secured to the first plastic link, and the coil spring biases the prosthetic knee toward an extended position. The polycentric prosthetic knee may include a locking mechanism that prevents rotation of the plastic lower member with respect to the plastic upper member. The locking mechanism may include a locking pin that resides in a first bore in the first plastic link and a second bore in the plastic upper member. Moving the locking pin from the second plastic bore permits rotation of the plastic lower member with respect to the plastic upper member. The plastic lower member may be adapted to couple to a pylon. A pylon bore in the plastic lower member may be adapted to receive an upper end of a pylon. A plurality of fastener bores in the plastic lower member may be substantially perpendicular to the pylon bore and may extend from an outer surface of the plastic lower member to the pylon bore. A plurality of fasteners may reside in the plurality of fastener bores and may be adapted to secure the upper end of the pylon in the pylon bore. The plurality of fasteners may be adapted to secure the upper end of the pylon in the pylon bore by applying pressure to the upper end of the pylon. The plurality of fasteners may be screws. The plurality of fastener bores may be evenly distributed about a circumference of the pylon bore. Two of the fastener bores may be parallel to the first lower axis and the second lower axis, and two of the fastener bores perpendicular to the first lower axis and the second lower axis. At least one of the plastic upper member, the plastic lower member, the first plastic link, the second plastic link, or the third plastic link may include a load-bearing plastic structure made of copolymer polyacetal plastic. In some cases, the polycentric prosthetic knee may meet or exceed testing standards for prosthetic knees set forth by the International Organization for Standardization (ISO), and in some cases, the polycentric prosthetic knee may not meet or exceed testing standards for prosthetic knees set forth by the ISO. The polycentric prosthetic knee may support an external static load of 4,480 Newtons.

In preferred embodiments the method may include using a drilling jig to manufacture component parts of a prosthetic knee. The drilling jig may include a housing and pressure members extending into cavities in the housing. The pressure members may be adapted to force a plastic block in each cavity toward a reference location in the cavity. The drilling jig may include drill-site bores extending from the outer surface of the housing to the cavities. The drill-site bores may be adapted to guide a drill bit into the cavities to drill bores through the block in the cavities. The bores drilled in the blocks may be adapted to carry rotational axes of a polycentric prosthetic knee. The bores may include a first bore adapted to carry a first rotational axis and a second bore adapted to carry a second rotational axis. The plurality of drill-sites may be adapted to guide the drill bit to form the plurality bores within a specified distance from each other. The plurality of drill-sites may be adapted to guide the drill bit to form the first plurality bores within a specified runout tolerance.

The details of one or more implementations are set forth in the accompanying drawings and the description below. Other features, objects, and advantages will be apparent from the description and drawings, and from the claims.

### DESCRIPTION OF DRAWINGS

FIG. 1A is a diagram of an example trans-femoral amputee with a lower-limb prosthesis.
FIG. 1B is a front view of an example prosthetic knee.
FIGS. 1C and 1D are perspective views of the example prosthetic knee 108a of FIG. 1B.
FIG. 1E is a perspective view of an example prosthetic knee that includes an example extension assist mechanism.
FIG. 1F is a side view of the example prosthetic knee 108a of FIG. 1B in an extended orientation.
FIG. 1G is a side view of the example prosthetic knee 108a of FIG. 1B in a flexed orientation.
FIG. 1H is a side view of an example prosthetic knee that includes an example extension assist mechanism.
FIG. 1I is a front view of the example prosthetic knee of FIG. 1H.
FIG. 1J is a side view of an example prosthetic knee that includes an example locking mechanism.
FIG. 2 is a plot showing the spatial coordinates of the instantaneous center of rotation of the example prosthetic knee 108a and 108b of FIGS. 1B-1G.
FIG. 3A is a perspective view of an example prosthetic knee.
FIG. 3B is a front view of the example prosthetic knee 300 of FIG. 3A.
FIG. 4A is a perspective view of an example prosthetic knee.
FIG. 4B is a front view of the example prosthetic knee 400 of FIG. 4A.
FIG. 5A is a perspective view of an example prosthetic knee.
FIG. 5B is a side view of the example prosthetic knee of FIG. 5A in an extended orientation.
FIG. 5C is a side view of the example prosthetic knee 500 of FIG. 5A in a flexed orientation.
FIG. 5D is a diagram showing internal features of the example prosthetic knee 500 of FIG. 5A.
FIG. 6A is a perspective view of an example prosthetic knee that includes an example extension assist mechanism.
FIG. 6B is a perspective view of the upper member 620 of the example prosthetic knee 600 of FIG. 6A.
FIG. 6C is a side view of the lower member 622 of the example prosthetic knee 600 of FIG. 6A.
FIG. 7 is a diagram of the example spring 550 of FIG. 5D.
FIG. 8 is a diagram showing an example technique for manufacturing a prosthetic knee.
FIGS. 9A-9E are diagrams showing an example technique for using a drilling jig to fabricate links of a prosthetic knee.
FIGS. 10A-10D are diagrams showing an example technique for using a drilling jig to fabricate components of a prosthetic knee.
FIG. 11 is a diagram showing example templates for fabricating components of a prosthetic knee.
FIG. 12A is a plan view of a cover component of an example drilling jig.
FIG. 12B is a side view of the cover component 1202 of FIG. 12A.
FIG. 13A and 13C are plan views of a slot component of an example drilling jig.
FIG. 13B is a cross-sectional view of the slot component 1302 of FIG. 13A.
FIG. 14 is a plan view of a cover component of an example drilling jig.
FIGS. 15A, 15B, and 15F are plan views of a slot component of an example drilling jig.
FIG. 15C is a side view of a first side of the slot component 1502 of FIG. 15A.
FIG. 15D is a side view of a second side of the slot component 1502 of FIG. 15A.
FIG. 15E is a side view of a third side of the slot component 1502 of FIG. 15A.
FIG. 15G is a side view of a fourth side of the slot component 1502 of FIG. 15A.
FIG. 15H is a cross-sectional view of the slot component 1502 of FIG. 15A.
FIG. 16A is a side view of an example prosthetic knee.
FIG. 16B is a front view of the example prosthetic knee 1600 of FIG. 16A.
FIG. 17 is a perspective view of aspects of an example prosthetic device.
FIG. 18 is a perspective view of aspects of an example prosthetic device.
FIGS. 19A, 19B, 19C, and 19D are diagrams of an example graphical manufacturing guide for manufacturing a polycentric prosthetic knee.

Like reference symbols in the various drawings indicate like elements.

### DETAILED DESCRIPTION

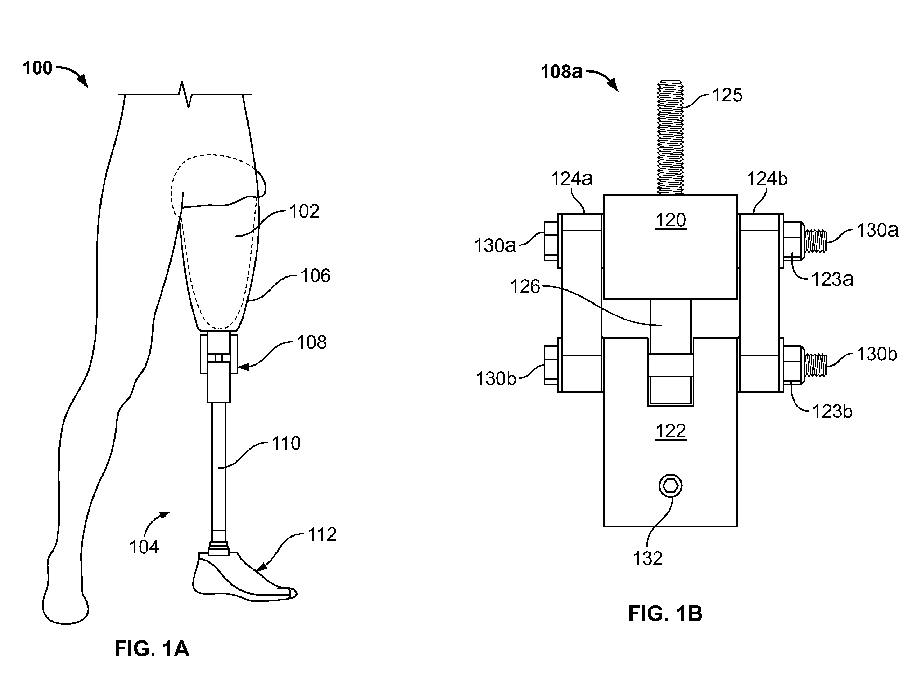

FIG. 1A is a diagram of an example transfemoral amputee 100 having a residual limb 102 that carries a lower-limb prosthesis 104. The example transfemoral amputee 100 is a human whose left leg has been amputated above the knee, resulting in the residual limb 102 that includes a portion of the amputee's thigh. Generally, a transfemoral amputee may have an amputated left leg, an amputated right leg, or both. As such, the lower-limb prosthesis 104 may be secured to a left residual limb or a right residual limb, or an amputee having both legs amputated may carry a prosthesis on each of the right and left residual limbs.

In some implementations, the prosthesis 104 includes features that allow the prosthesis 104 and/or one or more of its component parts to be safely and reliably manufactured, assembled, deployed, maintained, and/or repaired by laborers having simple skill sets, using common, inexpensive tools and materials. For example, one or more component parts of the prosthesis 104 may be manufactured without access to medical personnel having advanced training and/or without the need for advanced tools, materials, or manufacturing equipment. Such prosthetic devices may be beneficial for all transfemoral amputees, for example, by providing an inexpensive and largely maintenance-free prosthesis. Such prosthetic devices may be beneficial for amputees across the developing world, for example, in locations and conditions where certain types of medical care, manufacturing facilities, and/or material are not frequently accessible. The prosthesis 104 and/or its component parts may be designed in accordance with the manufacturing, rehabilitation and cultural constraints of various international sites.

The example prosthesis 104 shown includes a socket 106, a prosthetic knee 108, a pylon 110, and a prosthetic foot 112. A prosthesis may include additional and/or different features in the configuration shown and/or in a different configuration. In some implementations, the prosthesis 104 and/or one or more of its component parts can be used for a variety of different amputees and/or in a variety of different conditions with little or no modification. In some implementations, the prosthesis 104 and/or one or more of its component parts may include features that are adapted for the needs, preferences, and/or physical parameters of a particular amputee. For example, the prosthesis 104 may include features that allow the prosthesis 104 to function in wet environments, including water submersion conditions and/or frequent exposure to other types of fluids.

In some implementations, the prosthesis 104 can be used in a variety of different conditions without modification. For example, in some cases, the prosthesis 104 and/or its component parts may used for either a left residual limb or a right residual limb; in some cases, the prosthesis 104 and/or its component parts may be used by amputees having a variety of different heights, weights, builds, ages, gaits, weight distributions, strengths, and/or other physical parameters; in some cases, the prosthesis 104 and/or its component parts may be used by amputees using the knee for a variety of different speeds, distances, surface types, environments, and/or other types of usage conditions.

In some implementations, the prosthesis 104 may include features that are adapted for the needs, preferences, and/or physical parameters of a particular amputee. For example, in some cases, the prosthesis 104 and/or its component parts may be adapted for a left residual limb or a right residual limb; in some cases, the prosthesis 104 and/or any of its component parts may be adapted for a particular amputee's height, weight, build, age, gait, weight distribution, strength, and/or other physical parameters; in some cases, the prosthesis 104 and/or its component parts may be adapted for a particular amputee's typical movement speeds, distances, surface types, environments, and/or other types of usage conditions. In some implementations, the prosthesis 104 may include an exoskeleton. An exoskeleton may include an outer mechanical framework that supports and/or provides the described functionality of the components of the prosthesis. For example, the prosthetic knee 108 may be coupled to an exoskeleton that functions as a shank prosthesis. Aspects of an exoskeleton may be used in addition to, or as an alternative to, the pylon 110 and/or other components of the prosthesis 104.

The socket 106 provides the interface between the amputee 110 and the prosthesis 104. As shown in FIG. 1A, the socket 106 is secured to the residual limb 102 and to the prosthetic knee 108. The socket 106 transmits force between the residual limb 102 and the prosthesis 104. The socket 106 may include a fiberglass and polypropylene socket, an aluminum socket, and/or a socket made of other materials. The socket 106 may be based on any type of socket design, for example, the ischial containment socket design, the quadrilateral socket design, and/or other types of socket designs. In some implementations, the socket 106 is based on a plaster mold of the residual limb 102.

The socket 106 may be secured to the residual limb 102 using a suspension mechanism. Many different types of suspension mechanisms can be used. For example, the socket 106 may include self-suspension mechanism, a suction suspension mechanism, a harness suspension mechanism, and/or other types of suspension mechanisms. The suspension mechanisms may be adapted for the shape of the particular residual limb 102 and/or may be useable with residual limbs of different shapes and sizes. Suspension mechanisms may include liner materials (e.g., fabrics, gels, and/or others), elastic materials (e.g., neoprene, rubber, latex, and/or others), breathable materials, belts, cuffs, wedges, straps, sleeves, air valves, and/or other features.

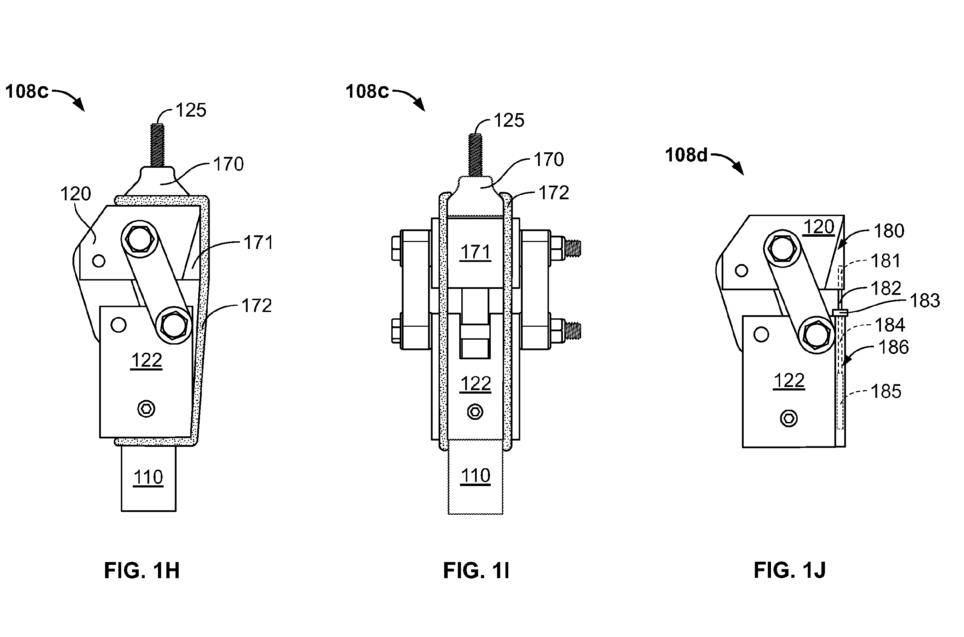

The socket 106 may be secured directly to the prosthetic knee 108, or the socket 106 may be secured to the prosthetic knee 108 by an adapter. For example, various types of adapters may be used to allow a prosthetic knee to mechanically couple with a variety of different types of socket. In such cases, the top of the prosthetic knee 108 may include a threaded bolt or another type of fastener that mechanically couples to a variety of different adapters, with each adapter designed for a different type of socket. A pyramid adapter and/or other types of adapters may be used to couple the prosthetic knee 108 to the socket 106. An example of a pyramid adapter 170 is shown in FIGS. 1H and 1I. In some implementations, a first adapter may be used to secure the knee 108 to fiberglass and polypropylene sockets, and a second adapter may be used to secure the knee 108 to aluminum sockets. The socket 106 may be secured directly to the prosthetic knee 108 in a variety of ways. In some implementations, the top of the prosthetic knee 108 includes a threaded member received in lower end of the socket 106. In some implementations, the top of the prosthetic knee 108 includes a bore that receives a threaded member on a lower end of the socket 106. The socket 106 may be secured to the prosthetic knee 108 by screws, bolts, pins, adhesives, clamps, grooves, threading and/or other types of fastening members and materials.

The prosthetic knee 108 provides a bending motion of the prosthesis 104. Additional and/or different bending or flexing motion may be provided by other components of the prosthesis 104, for example, the prosthetic foot 112, a prosthetic ankle, and/or others. The prosthetic knee 108 has an extended position corresponding to an extended position of the prosthesis 104. When the prosthesis 104 is in its extended position, the pylon 110 is substantially aligned with the residual limb 102. The prosthesis 104 in its extended position can support the weight of the amputee 100. The prosthetic knee 108 has a range of flexed positions corresponding to a range motion of the prosthesis 104. The range of motion corresponds to a range of angles between the pylon 110 and the residual limb 102. The prosthesis 104 traverses at least a portion of the range motion during the swing phase of the amputee's walking motion. The prosthetic knee 108 may allow the prosthesis 104 to flex more than ninety degrees from the extended position. For example, prosthetic knee 108 may allow the prosthesis 104 to flex up to 136 degrees or another angle from the extended position. In some implementations, the prosthetic knee 108 can flex up to 180 degrees from the extended position. Flexation of the prosthetic knee 108 may allow the prosthesis 104 to accommodate the amputee 100 sitting, kneeling, standing, walking, jogging, swimming, and/or performing other functions.

The example prosthetic knee 108 is an external, polycentric, four-bar joint design. The prosthetic knee 108 is external, in that the joint is designed for external application to a residual limb, rather than internal (e.g., surgical) implantation within the body. The four-bar joint design includes four mechanical axes that prescribe the bending motion of the knee. As a polycentric joint, the prosthetic knee 108 has an instantaneous center of rotation that moves with respect to the mechanical axes during at least part of the bending motion of the knee. A polycentric knee design may provide the prosthesis 104 with improved ground clearance during the swing phase, a more stable extended position, and/or other advantages. For example, the polycentric design may cause the length of the prosthesis 104 to become shorter when the prosthetic knee 108 is flexed, which may help the toe of the prosthetic foot 112 avoid striking the walking surface as the prosthesis 104 is extended. A polycentric knee design may allow the prosthesis 104 to be used with greater ease. For example, the polycentric knee design may provide a more stable extended position and/or require less energy input from the amputee 100 during use. A polycentric knee design may allow the prosthesis 104 to be used in more versatile conditions. For example, the polycentric knee design may allow safer and/or more reliable operation on inclined surfaces and/or in other environments.

The prosthetic knee 108 may be implemented as a low-cost, durable prosthetic joint, and may include interchangeable parts that can be easily and inexpensively replaced. For example, the prosthetic knee 108 and its component parts may be safely and reliably manufactured, assembled, deployed, maintained, and/or repaired by laborers having simple skill sets, using common, inexpensive tools and materials. The geometric design of the prosthetic knee 108 may allow manufacturing tolerances that can be achieved without highly skilled technicians and/or without computer-guided manufacturing processes. For example, the dimensions (e.g., length, width, height, etc.) of the knee components may have sufficiently high tolerances that the prosthetic knee 108 can be reliably manufactured and/or repaired by tools and/or in conditions that do not guarantee extremely high precision and accuracy.

In some implementations, the prosthetic knee 108 may include parts that can be manufactured and/or purchased in diverse international locations. For example, the structural components may be made of solid plastic material (e.g., DuPont's Delrin and/or another type of plastic material) that can be easily found, salvaged, and/or purchased in a variety of locations. As another example, the fasteners and other components may include nuts, bolts, pins, screws, washers, and/or other materials that can be found at typical hardware stores, or may be salvaged or purchased in a variety of locations. The particular materials and component parts included in the prosthetic knee 108 may be inexpensive and/or accessible around the world, including most underdeveloped nations

In some implementations, the prosthetic knee 108 includes parts that can be shaped, manipulated and/or adjusted by simple mechanical tools and/or inexpensive power tools. For example, the structural components may be made of solid plastic material (e.g., DuPont's Delrin and/or another type of plastic material) that can be cut, drilled, sanded, and/or otherwise manipulated using readily-available tools. In some implementations, parts of the prosthetic knee 108 can be cut using a hack saw, a band saw and/or another type of saw, parts of the prosthetic knee 108 can be drilled using a hand-held drill, a drill press, and/or another type of drill, and/or parts of the prosthetic knee 108 that can be sanded by hand and/or using a sanding machine.

In some implementations, the prosthetic knee 108 may include parts that are not available throughout the world. For example, the prosthetic knee 108 may include materials that are shaped and/or adjusted by specialized equipment. The structural components of the prosthetic knee 108 may include materials manufactured by automated machining, casting, and/or molding processes. For example, the prosthetic knee 108 may include component parts manufactured by a computed numerically controlled (CNC) process, compression molding process, transfer molding process, injection molding process, laser cutting process, mechanical cutting process, melting or fusion process, and/or other types of manufacturing processes. The structural components of the prosthetic knee 108 may include materials selected for durability, weight, strength, appearance, profile, and/or other functional and cosmetic factors..

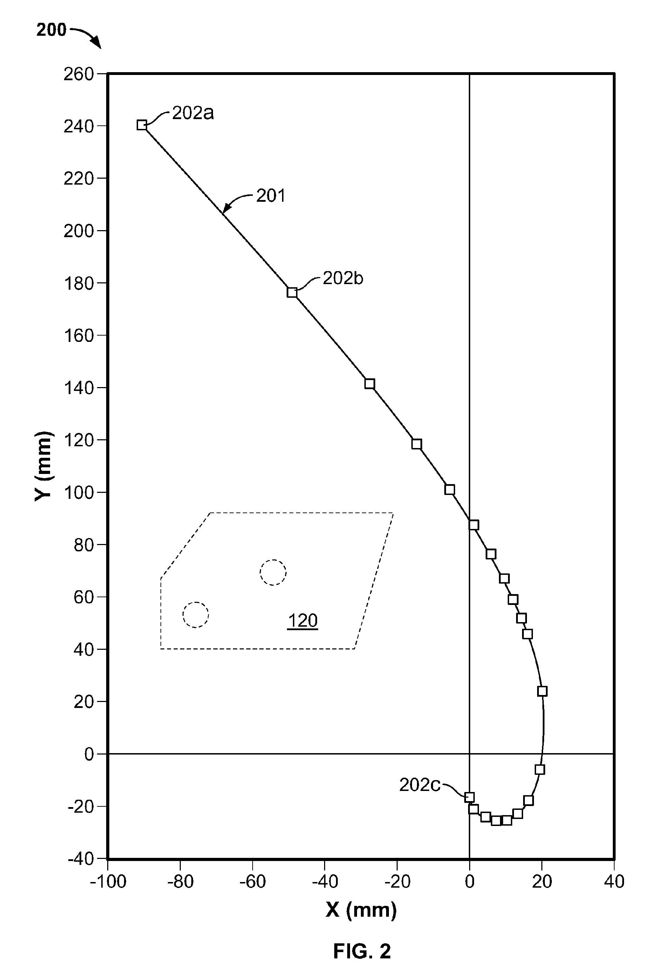

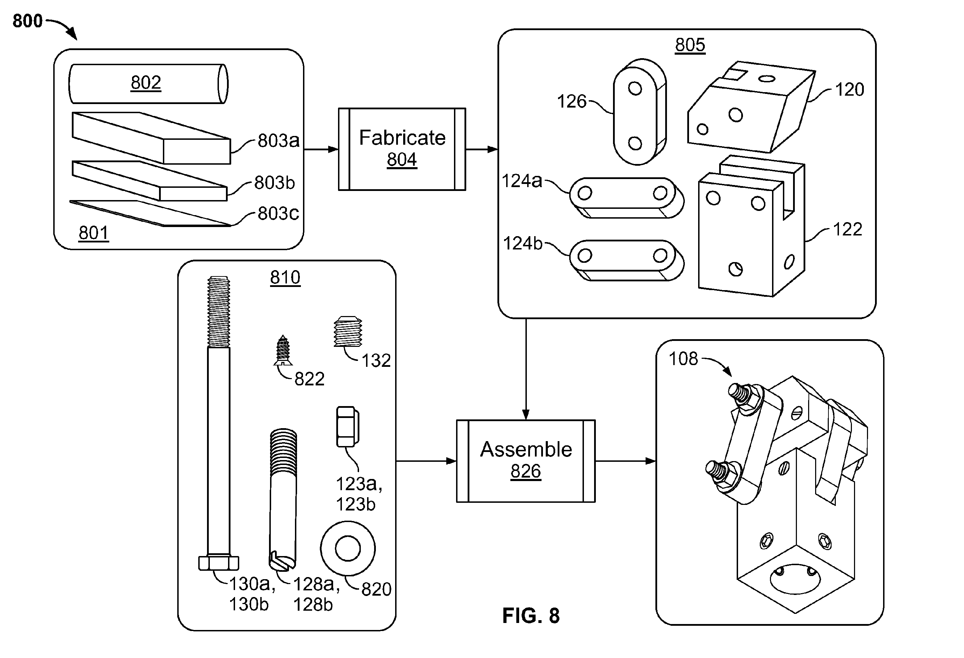

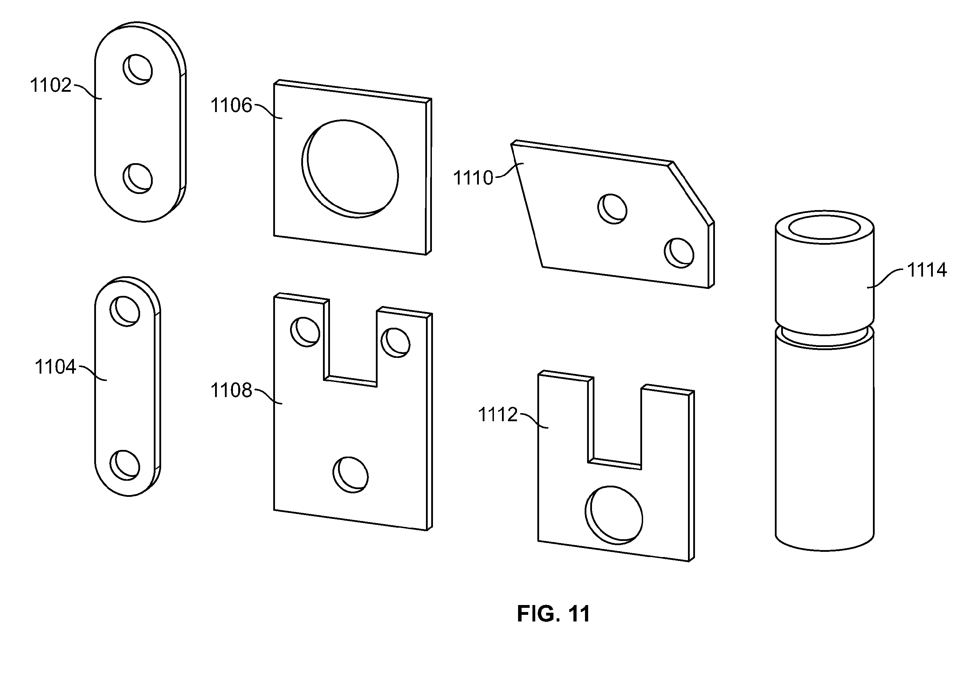

The example prosthetic knee 108 may be implemented using a variety of different prosthetic knee designs. For example the prosthetic knee 108 may be implemented based on any of the example designs shown in FIGS. 1B-1J, 3A, 3B, 4A, 4B, 5A-5D, and 6A-6C, and/or the prosthetic knee 108 may be implemented based on a different design. The prosthetic knee 108 may have an instantaneous center of rotation that moves as shown in FIG. 2 during the bending motion of the knee, or prosthetic knee 108 may have an instantaneous center of rotation that moves in a different manner during the bending motion of the knee. The prosthetic knee may be made according to the example techniques shown in FIGS. 8, 9A-9E, 10A-10D, and/or according to different or additional techniques. The prosthetic knee 108 may be made using the example equipment shown in FIGS. 11, 12A, 12B, 13A-13C, 14, 15A-15H, and/or the prosthetic knee 108 may be made using additional or different equipment.

The pylon 110 is secured to the prosthetic knee 108 and the prosthetic foot 112. The pylon 110 may be implemented as a rigid structure that transfers force from the prosthetic foot 112 to the prosthetic knee 108. The pylon 110 may be made of metal, fiberglass, plastic, wood, and/or combinations of these and other types of materials. The pylon 110 may include a cosmesis that emulates the appearance of a lower leg. For example, the cosmesis may include the geometry of a calf muscle, the cosmesis may have a color tone and/or texture that emulates the skin of the amputee 100, and/or the cosmesis may include other features that give the pylon 110 a natural appearance, size, and shape. The cosmesis may be made of foam, rubber, plastic, wood, and/or other materials.

The pylon 110 may be secured directly to the prosthetic knee 108, or the pylon 110 may be secured to the prosthetic knee 108 by an adapter. For example, various types of adapters may be used to allow a single prosthetic knee to mechanically couple with a variety of different types of pylon. Example adapters include pyramid adapters and adapters of other types. The bottom of the prosthetic knee 108 may include a bore or a fastener that mechanically couples to a variety of different adapters, with each adapter designed for a different type of pylon. The pylon 110 may be secured directly to the prosthetic knee 108 in a variety of ways. In some implementations, the bottom of the prosthetic knee 108 includes a threaded member received in the upper end of the pylon 110. In some implementations, the bottom of the prosthetic knee 108 includes a bore that receives a threaded member on an upper end of the pylon 110. The pylon 110 may be secured to the prosthetic knee 108 by screws, bolts, pins, adhesives, clamps, grooves, threading and/or other types of fastening members and materials. In some implementations, an upper end of the pylon 110 is secured in a lower bore of the prosthetic knee 108 by four fasteners extending into the lower bore and engaging the pylon 110.

The prosthetic foot 112 is secured to the pylon 110. When the amputee 100 is walking or standing, the bottom of the prosthetic foot 112 contacts the walking or standing surface and transfers force from the surface to the prosthesis 104. The pylon 110 may be secured directly to the prosthetic foot 112, or the pylon 110 may be secured to the prosthetic foot 112 by an adapter, an ankle joint, or another type of device. The ankle joint, adapter, and/or other device may include any type of device that mechanically couples the prosthetic foot 112 to the pylon 110. In some implementations the prosthetic foot 112 may be interchangeable with other prosthetic devices and/or adapted for a special purpose. For example, the prosthetic foot 112 may be adapted for walking, running, swimming, and/or performing other functions. In some implementations, the prosthetic foot 112 and the pylon 110 may be detachable from the prosthetic knee 108. In some implementations, the prosthetic foot 112 may be integrally formed with the pylon 110. The prosthetic foot 112 may carry a shoe.

The prosthesis 104 may include additional components and/or features not shown. The prosthesis 104 may include adaptors, fasteners, and/or other features that allow the various components to be secured to one another and/or interchanged with other parts. For example, the prosthesis 104 may include an adaptor that allows the prosthetic knee 108 to be secured to an aluminum socket, a fiberglass socket, and/or another type of socket; the prosthesis 104 may include an adaptor that allows the prosthetic knee 108 to be secured to an aluminum pylon, a fiberglass pylon, and/or another type of pylon. The prosthesis 104 may include cosmetic parts affecting the appearance of the prosthesis 104 and/or its component parts. For example, the prosthesis 104 may include a cosmesis covering the socket 106, the knee 108, the pylon 110, the foot 112 and/or other features to emulate the appearance (e.g., skin, muscles, hair, and/or others) of a lower leg. The prosthesis 104 may include additional prosthetic joints and/or changeable or adjustable features that allow enhanced functionality. For example, the prosthesis 104 may include a prosthetic ankle that allows flexation of the prosthetic foot 112 with respect to the pylon 110; the prosthesis 104 may include one or more locking mechanisms that allow the prosthesis 104 to be secured at a particular angle, orientation, and/or position; the prosthesis 104 may include one or more release mechanisms that allows the prosthesis 104 and/or its component parts to be removed; the prosthesis 104 may include one or more energy-storing or energy-generating mechanisms that provide energy for operation of the prosthesis 104. The prosthesis may include features that enhance comfort for the amputee 100. For example, the socket 106 may include padding and/or adjustable features.

In some implementations, the prosthetic knee 108 is a plastic knee that meets standards for lower limb prosthetic devices set forth by the International Organization for Standardization (ISO). Implementations of the prosthetic knee 108 where the upper member 120, the lower member 122, the two anterior links 124a, 124b, and the posterior link 126 are made of plastic material and assembled using common hardware have been tested and found to meet or exceed the standards set forth in ISO 10328, First Edition, dated October 1, 2006, entitled "Prosthetics -- Structural testing of lower-limb prostheses -- Requirements and test methods." The standards include, for example, fatigue tests where the prosthetic knee is cycled through a time-varying external load, torsional strength tests where an external torsional force is applied to the prosthetic knee, and static loading tests where a static external load is applied to the prosthetic knee. All tests were performed under the conditions and criteria set forth the ISO 10328 referenced above.

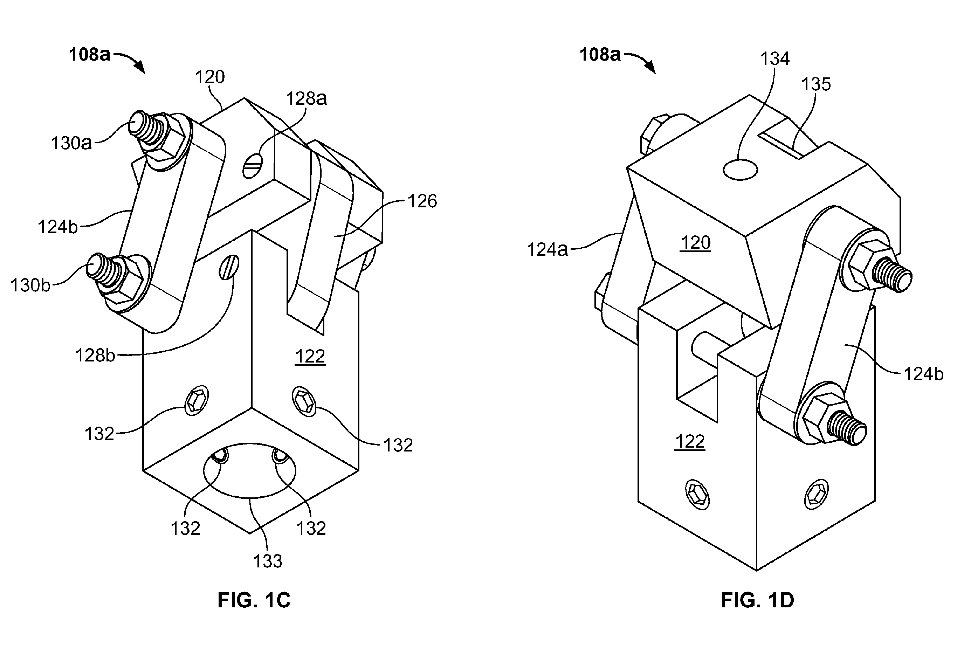

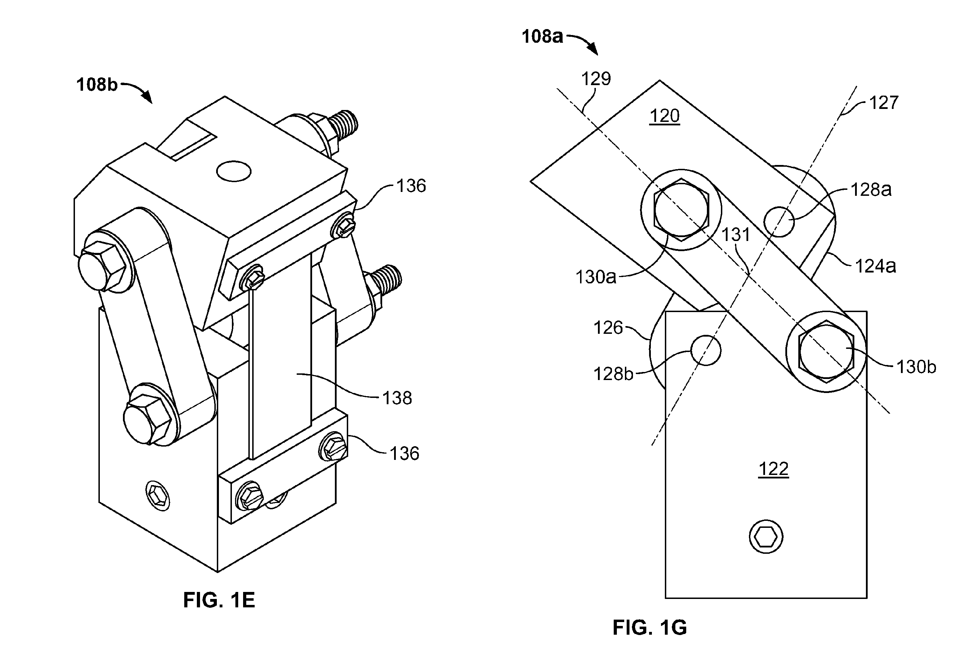

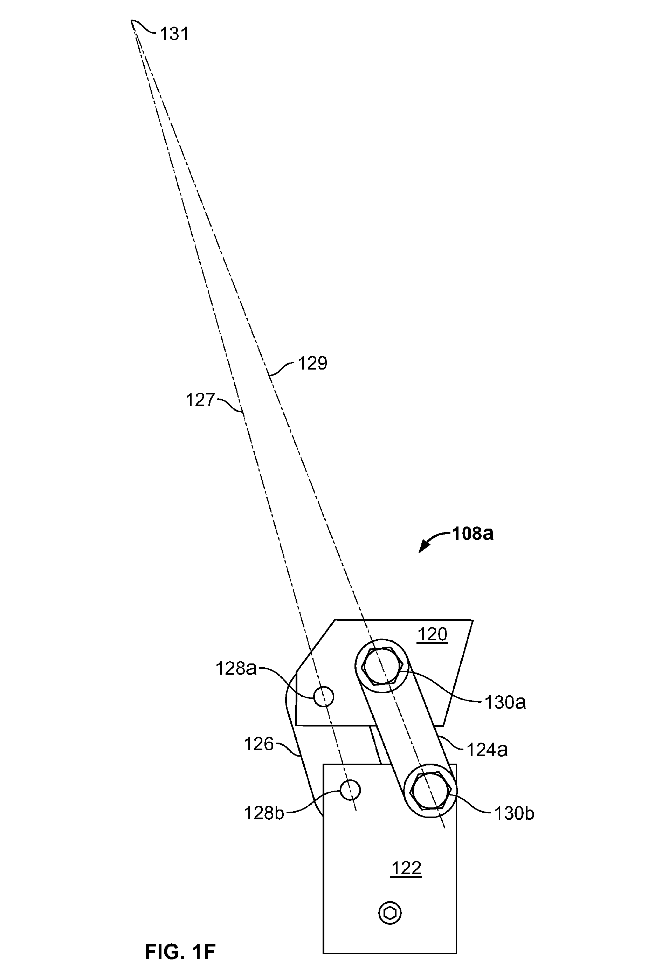

In particular, the embodiment of the prosthetic knee 108a shown in FIGS. 1A, 1B, 1C, 1D, 1F, and 1G was tested and found to meet or exceed the requirements of ISO 10328, First Edition, dated October 1, 2006, entitled "Prosthetics -- Structural testing of lower-limb prostheses -- Requirements and test methods." Multiple samples of this embodiment were tested and all samples met or exceeded the requirements set forth in the standard. The tested embodiment of the prosthetic knee included an upper member, lower member, two anterior links and a posterior link all made of acetal homopolymer plastic. The upper member and the lower member were fabricated from 60mm round stock source material. The links were fabricated from 16mm flat stock source material. The mechanical axes of rotation were stainless steel hex bolts secured to the upper member and the lower member with locking hex nuts. The stainless steel hex bolts forming the upper and lower posterior axes were shortened to the approximate width of the lower member and the upper members (approximately 50mm). The prosthetic knee was tested with a pyramid adapter, as shown, for example, in FIGS. 1J and 1I. The tests performed include several load profiles, cycle frequencies, fixtures, offsets, distances, and other test conditions set forth in the ISO standard. The prosthetic knee was tested at multiple load levels and in multiple conditions. In an example test, the prosthetic knee in the extended position supported an external load of 4,480 Newtons. The external load was applied in the manner specified by the ISO standard. In another example test, the external load applied to the prosthetic knee was varied over time in a cylical manner, as specified by the ISO standard; the load profile included 3,000,000 cycles at a frequency of 0.89 Hz; the maximum load for each cycle was 1,330 Newtons; the mean load over each cycle was 690 Newtons; the load varied over each cycle by as much as 640 Newtons; after the load cycling a final static force of 2,240 Newtons was applied to the knee. In another example test, the prosthetic knee withstood torsional forces of up to 50 Newton-meters. Other embodiments of the example prosthetic knees described herein may also meet or exceed the ISO 10328 standards, including the tests described above.

FIG. 1B is a front view of an example prosthetic knee 108a. Alternative views of the example prosthetic knee 108a are shown in FIGS. 1B, 1C, 1F, and 1G. FIG. 1C is a perspective view showing aspects of the lower and posterior portions of the example prosthetic knee 108a; FIG. 1D is another perspective view showing aspects of the upper and anterior portions of the example prosthetic knee 108a. FIG. 1F is a side view of the example prosthetic knee 108a in an extended position, and FIG. 1G is a side view of the example prosthetic knee 108a in a flexed position. FIG. 1E is a perspective view of an example prosthetic knee 108b, which is a version of the prosthetic knee 108a that has been modified to include an example extension assist mechanism. In some cases, the prosthetic knee 108a may be modified or adapted to include one or more features of the example prosthetic knees shown in FIGS. 1E, 1H-1J, 3A-3B, 4A-4B, 5A-5D, 6A-6C, and 16A-16B, and/or other types of prosthetic knees.

The example prosthetic knee 108a shown in FIGS. 1B, 1C, 1E, 1F, and 1G includes an upper member 120, a lower member 122, two anterior links 124a, 124b, and a posterior link 126. The upper member 120, the lower member 122, the two anterior links 124a, 124b, and the posterior link 126 may be made of the same material. In the example shown, the upper member 120, the lower member 122, the two anterior links 124a, 124b, and the posterior link 126 are all made of Delrin, produced by DuPont. In some implementations, the upper member 120 and the lower member 122 can be made from 65 mm Diameter Delrin rod and/or 50 mm Delrin sheet, and the anterior links 124a, 124b and the posterior link 126 can be made from 16 mm Delrin sheet. One or more of the components may be made from round stock, flat stock, and/or different types of source materials, which may have different dimensions and geometries.

The upper member 120, the lower member 122, the two anterior links 124a, 124b, and the posterior link 126 may be made of one or more of a variety of different plastic materials. For example, one or more of the components may be made of plastics (e.g., Delrin, and/or other types of plastic). The upper member 120, the lower member 122, the two anterior links 124a, 124b, and the posterior link 126 may be manufactured according to the techniques shown in FIGS. 8, 9A-9E, 10A-10D, and/or according to different or additional techniques. The upper member 120, the lower member 122, the two anterior links 124a, 124b, and the posterior link 126 may be manufactured using the equipment shown in FIGS. 11, 12A, 12B, 13A-13C, 14, 15A-15H, and/or the prosthetic knee 108 may be made using additional or different equipment.

The example prosthetic knee 108a includes four axes of mechanical rotation: an upper posterior axis, a lower posterior axis, an upper anterior axis, and a lower anterior axis. In the example shown, the upper posterior axis is formed by a pin 128a, the lower posterior rotational axis is formed by a pin 128b, the upper anterior axis is formed by a bolt 130a, the lower anterior rotational axis is formed by a bolt 130b. The bolts can be secured by nuts and/or other suitable hardware. Additional and/or different types of devices and/or hardware may be used to form the axes of mechanical rotation. For example, any suitable type of bolt, screw, pin, rod, bar, standoff, or similar device may be used to form any of the mechanical axes of rotation. The anterior links 124a, 124b and the posterior link 126 can rotate on the mechanical axes. When the prosthetic knee 400 flexes or extends, the lower member 122 rotates with respect to the upper member 120 about an instantaneous center of rotation defined by the upper posterior axis, the lower posterior axis, the upper anterior axis, and the lower anterior axis. For example, the instantaneous center of rotation is defined by the mechanical axes of rotation as shown in FIGS. 1G and 1H, and the instantaneous center of rotation may move during the flexing or bending motion as shown in FIG. 2.

In the example shown, the bolt 130a and a nut 123a secure the anterior links 124a, 124b to the upper member 120; the bolt 130b and a nut 123b secure the anterior links 124a, 124b to the lower member 122. The bolt 130a may carry washers and/or other hardware to reduce friction between the anterior links 124a, 124b and the upper member 120; the bolt 130b may carry washers and/or other hardware to reduce friction between the anterior links 124a, 124b and the lower member 122. (Hardware are described with reference to well-known ISO metric standards). Bolts, washers, and nuts of other types and sizes may be used. In some implementations, the washers may include plastic washers, metal washers and/or washers made of different materials. One or more of the washers may be omitted or incorporated into another component of the knee. For example, the upper member 120, the lower member 122, the anterior links 124a, 124b, and/or the posterior link 126 may be manufactured with one or more washers or spaces built in.

On the bolt 130a forming the upper anterior axis, washers can be positioned between the bolt head and the anterior link 124a, between the anterior link 124a and the upper member 120, between the upper member 120 and the anterior link 124b, and/or between the anterior link 124b and the nut 123a. On the bolt 130b forming the lower anterior axis, washers can be positioned between the bolt head and the anterior link 124a, between the anterior link 124a and the lower member 122, between the lower member 122 and the anterior link 124b, and/or between the anterior link 124b and the nut 123b. On the pin 128a forming the upper posterior axis, washers can be positions on both sides of the posterior link between the posterior link and the upper member 120. On the pin 128b forming the lower posterior axis, washers can be positions on both sides of the posterior link between the posterior link and the lower member 122.

In some example implementations, the bolts 130a, 130b are M8 x 1.25 x 100 mm bolts that each carry four M8 washers, and the nuts 123a, 123b are M8 nylon lock nuts. In some example implementations, the pins 128a, 128b are modified M8 x 1.25 x 60 mm bolts. In such example implementations, the head ends of two M8 x 1.25 x 60 mm bolts are cut off to form two M8 x 1.25 threaded pins 128a, 128b having a length approximately equal to the width of the upper member 120 and the lower member 122. In such example implementations, a saw or another cutting device may be used to form a head in the ends of each pin 128a, 128b, so that the pins 128a, 128b may be driven into the upper member 120 and the lower member 122 *(e.g.,* using a screwdriver, or a similar device).

In the example prosthetic knee 108a, the upper member 120 includes a bore 134 in its upper surface, as shown in FIG. 1D. The bore 134 may terminate at a depth in the upper member 120, or the bore 134 may extend completely through the upper member 120. In some implementations, a bolt 125 or another type of fastener extends through the bore 134, as shown in FIG. 1B. The bolt 125 may secure the prosthetic knee 108a to a socket, and/or the bolt may carry an adapter that secures the prosthetic knee 108a to a socket. The bolt 125 can be a M10 x 1.5 x 50 mm bolt, or another size or type of bolt.

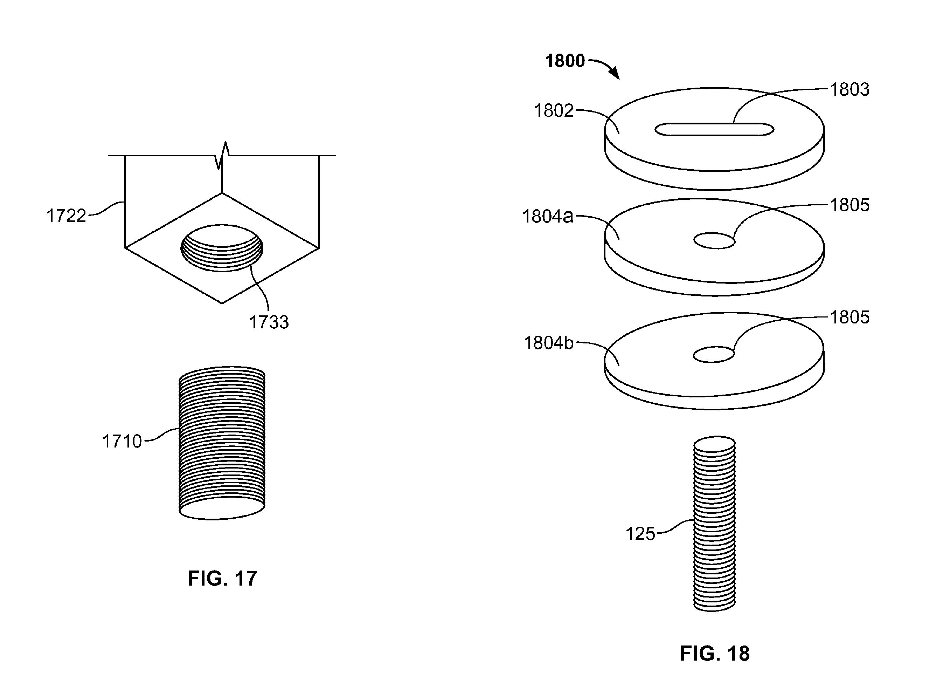

FIG. 18 is a perspective view of a mechanism 1800 that may be carried on the bolt 125 or on another type of fastener extending from the example prosthetic knee 108a or another type prosthetic knee. The mechanism 1800 enables adjustment of the mechanical coupling between the prosthetic knee and a socket 106. The mechanism 1800 may allow the angular alignment of the prosthetic knee and the socket to be adjusted. The mechanism 1800 may allow the placement of the prosthetic knee on the socket to be adjusted. The mechanism 1800 may allow additional and/or different aspects of coupling to be adjusted.

The mechanism 1800 includes an upper plate 1802, and two wedges 1804a, 1804b that are carried on the bolt 125. In some implementations, a different number of wedges are used *(e.g.,* 1, 3, 4, etc.). The upper plate 1802 and the wedges 1804a, 1804b may be made of metal, wood, plastic, and/or other materials. In an example implementation, the upper plate 1802 is made of metal and the wedges 1804a, 1804b are made of Delrin plastic. The upper plate 1802 includes an elongate slot 1803. The wedges 1804a, 1804b each include a bore 1805. The mechanism 1800 is shown in an exploded view in FIG. 18 for purposes of illustration. When the mechanism 1800 is installed in a prosthesis, the bolt 125 is inserted through the bores 1805 in the wedges 1804a, 1804b and through the slot 1803 in the upper plate 1802, and the two wedges 1804a, 1804b and the upper plate 1802 are stacked on top of the upper member.

The elongate slot 1803 in the upper plate 1802 allows the prosthetic knee to be shifted forward (in the anterior direction) or backward (in the posterior direction) on the socket. The anterior / posterior adjustment can affect stability of the prosthetic knee. For example, shifting the prosthetic knee anterior with respect to the socket shifts the instantaneous center of rotation anterior, which may reduce stability and/or allow easier flexation of the prosthetic knee in the extended position. As another example, shifting the prosthetic knee posterior with respect to the socket shifts the instantaneous center of rotation posterior, which may improve stability of the prosthetic knee and/or require greater lateral forces for flexation of the prosthetic knee. The wedges 1804a, 1804b allow the angular alignment between the prosthetic knee and the socket to be adjusted. For example, rotation of either of the wedges with respect to the other wedge and/or rotation of the wedges with respect to the prosthetic knee and the upper plate 1802 may adjust the angular alignment of the prosthetic knee and the socket. The mechanism 1800 may be used alone or in combination with other types of adapters and similar devices.

In the example prosthetic knee 108a, the upper member 120 includes a rubber stop 135, as shown in FIG. 1D. The posterior link 126 may contact the rubber stop 135 when the prosthetic knee 108a is fully extended, and the rubber stop 135 may absorb the impact of the posterior link 126. The rubber stop 135 may be cut from a sheet of rubber to the appropriate size and shape using a saw, scissors, and/or another type of cutting device. The rubber stop 135 may be secured to the upper member by a screw, nail, tack, adhesive, and/or another type of fastener. In an example implementation, the rubber stop 135 is 2 mm thick and is secured to the upper member 120 by a 2.9 mm self-tapping screw.

In the example prosthetic knee 108a, the lower member 122 includes a bore 133 in its lower surface, as shown in FIG. 1C. The bore 133 may terminate at a depth in the lower member 122. In some implementations, an end of a pylon or a pylon adapter resides in the bore 133. The pylon or pylon adapter may be secured in the bore 133 by threading, bolts, screws, pins, adhesive, and/or in another manner. The radius of the bore 133 may be sized to match the outer radius of the pylon. In some implementations, the radius of the bore 133 is larger than the radius of the pylon to allow for adjustment. For example, the radius of the bore 133 may be large enough to allow alignment of the pylon with the lower knee member 122 to be adjusted. In some implementations, the bore 133 can be tightened or loosened. For example, the circumference of the bore 133 may include a gap that can be tightened or loosened about the upper end of the pylon. In some implementations, the gap in the circumference of the bore 133 can be adjusted by manipulating a bolt or another type of fastener that causes the bore 133 to dilate or restrict.

In the example prosthetic knee 108a, the lower member 122 includes a screw 132 in each of the four faces of the lower member. The lower member 122 includes a first fastener 132 in the front of the lower member, second and third fasteners 132 in the sides of the lower member, and a fourth fastener 132 in the back of the lower member. The screws may extend to the interior of the bore 133 to secure the pylon or pylon adapter in the bore 133. Thus, the pylon may be secured in the bore 133 by the fasteners extending into the bore 133. In an example implementation, the fasteners 132 are M10 x 1.5 x 10 mm set screws that can be driven by a 5mm Allen key. The fasteners 132 may include bolts, pins, and/or other types of fasteners. The fasteners 132 extend through fastener bores that extend from an outer surface of the lower member 122 to the bore 133. The fastener bores are evenly distributed around the circumference of the bore 133. Two of the fastener bores are parallel to the mechanical axes of rotation (formed by the bolts 130a, 130b and pins 128a, 128b), and two of the fastener bores are perpendicular to the mechanical axes of rotation.

In the example prosthetic knees shown and described herein, the lower member may include a different number of fasteners and/or fastener bores for securing a pylon in the lower bore of the lower member. One or more of the fasteners and/or fastener bores may be located in additional and/or different circumferential and/or axial positions with respect to the lower bore. As such, in some implementations, the fasteners 132 of the example prosthetic knee 108a may be distributed unevenly about the circumference of the bore 133; fewer or more than four fasteners 132 may be used; and fasteners 132 having additional and/or different orientations and positions may be used.

FIG. 17 is a perspective view of an example prosthetic device that utilizes a threaded coupling between the pylon and the lower member of a prosthetic knee. FIG. 17 shows a lower member 1722 of a prosthetic knee, which may include features of the lower member 122 of the prosthetic knee 108a and/or features of other prosthetic knees shown and described. The lower member 1722 includes a threaded lower bore 1733 adapted to receive an upper end of a pylon 1710 of a prosthesis. The threading on the pylon 1710 may extend the full length of the pylon 1710, as shown in FIG. 17; or the threading may extend over a first portion of the pylon, with a second portion of the pylon having a non-threaded surface. The pylon 1710 shown in FIG. 17 may include features of the pylon 110 of the prosthesis 104; the pylon 1710 may include additional and/or different features. The upper end of the pylon 1710 can be inserted in the bore 1733 and secured in the bore by threading on the pylon 1710 coupling with the threading in the bore 1733. Thus, the pylon 1710 can be secured in the bore 1733 by the threaded coupling. In some implementations, fasteners and/or other devices are used as an alternative to and/or in addition to the threaded coupling.

The example prosthetic knee 108a is adapted to bear an external load applied to the plastic upper member 120 and/or the plastic lower member 122 when the polycentric prosthetic knee is in an extended position. The upper member 120 may receive an external load from a socket, a socket adapter, or another device secured to the upper member 120. A plastic structure of the upper member 120 is adapted to transfer the external load to the upper axes (128a, 130a). The upper axes (128a, 130a) are adapted to transfer the external load from the upper member 120 to the links (124a, 124b, 126). A plastic structure of the links (124a, 124b, 126) is adapted to transfer the external load from the upper axes (128a, 130a) to the lower axes (128b, 130b). The lower axes (128b, 130b) are adapted to transfer the external load from the links (124a, 124b, 126) to the lower member 120. A plastic structure of the lower member 120 is adapted to transfer the load from the lower axes (128b, 130b), for example, to a pylon secured in the bore 133.

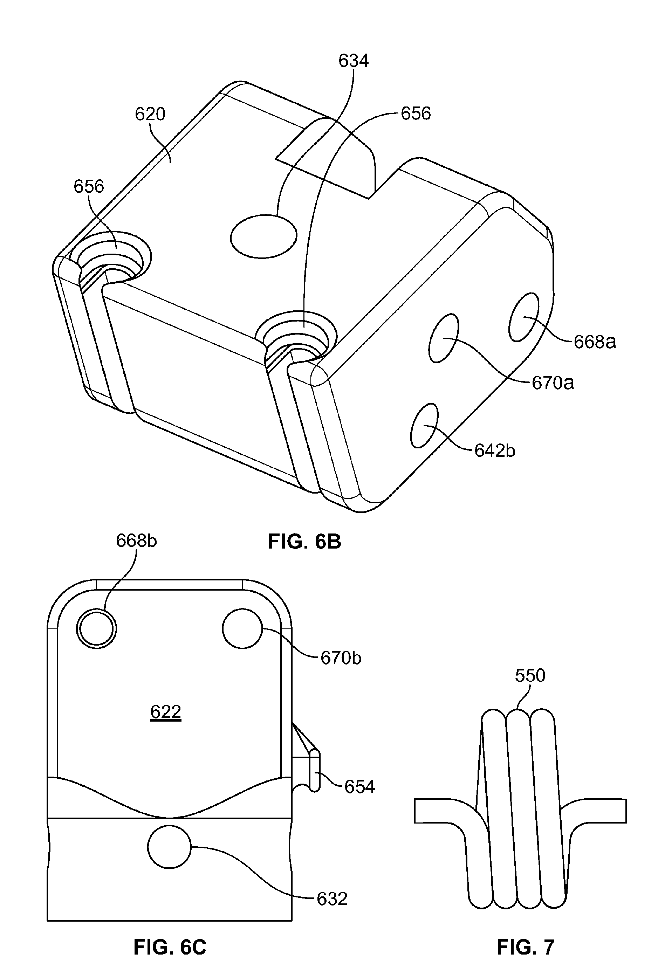

The example prosthetic knee 108b shown in FIG. 1E includes the features of the example prosthetic knee 108a shown in FIGS. 1B, 1C, 1E, 1F, and 1G. The prosthetic knee 108b additionally includes an extension assist mechanism. Extension assist mechanisms can be implemented in a variety of ways using different types of devices. An extension assist mechanism includes an energy storing mechanism that provides energy to assist with extending the prosthetic knee, and/or to assist with keeping the prosthetic knee in a stable extended position. Example energy storing mechanisms include springs, batteries, capacitors, flywheels, magnetized objects, compressed gas chambers, and others. Springs may include coils (*see, e.g.,* FIG. 7), bands, tension springs, extension springs, compression springs, torsional springs, and/or combinations of these and similar devices.

The example extension assist mechanism shown in FIG. 1E includes a rubber band 138 having a first end secured to the upper member 120 and a second end secured to the lower member 122. A first plate 136 screwed to an anterior face of the upper member 120 secures the first end of the rubber band 138 to the upper member 120 and a second plate 136 screwed to an anterior face of the lower member 122 secures the second end of the rubber band 138 to the lower member 122. The rubber band 138 may be secured to the upper member 120 and the lower member 122 in another manner. The plates 136 may be secured to the upper member 120 and the lower member 122 by any type of fastener, such as a screw, a bolt, an adhesive, a snap, and/or others. Friction between the rubber band 138 and both the upper member 120 and the plate 136 may prevent the first end of the rubber band 138 from slipping. Similarly, friction between the rubber band 138 and both the lower member 122 and the plate 136 may prevent the second end of the rubber band 138 from slipping. The rubber band 138 may be removable, for example, by loosening and/or removing the plates 136.

In the example shown, the rubber band 138 has a slack length, and when the rubber band 138 is stretched from its slack length, the rubber band 138 tends to contract back to its slack length, which may exert a force. As such, stretching the rubber band 138 from its slack length causes the rubber band 138 to store energy. When the prosthetic knee 108b is in the extended position, as shown in FIG. 1D, the rubber band 138 may be at its slack length, so that the rubber band 138 does not apply a force to the prosthetic knee 108b when the prosthetic knee 108b is in the extended position. Alternatively, when the prosthetic knee 108b is in the extended position, as shown in FIG. 1D, the rubber band 138 may be stretched from its slack length, so that the rubber band 138 applies a force to the prosthetic knee 108b when the prosthetic knee 108b is in the extended position. The force applied by the rubber band 138 when the prosthetic knee 108b is in the extended position may tend to hold the prosthetic knee 108b in the extended position.

During at least a portion of the bending motion of the prosthetic knee 108b, the rubber band 138 is stretched from its slack length. In various implementations, the rubber band 138 is stretched from its slack length immediately as the prosthetic knee 108b begins to flex, before the prosthetic knee 108b begins to flex, or after before the prosthetic knee 108b begins to flex. In some implementations, the prosthetic knee 108b bends through an initial angle (*e.g.,* 1 degree, 5 degrees, 15 degrees, or another angle) before the rubber band 138 is stretched from its slack length. When the rubber band 138 begins to stretch from its slack length, the rubber band 138 applies a force to the upper member 120 and to the lower member 122. The force applied by the rubber band 138 tends to direct the upper member 120 and lower member 122 toward the extended position of the prosthetic knee 108b.

In the example shown, after the rubber band begins to stretch from its slack length, the force applied by the rubber band 138 increases as the prosthetic knee 108b flexes away from the extended position. That is to say, the force applied by the rubber band 138 is at a maximum when the prosthetic knee 108b is fully flexed, for example, as the prosthetic knee 108a in FIG. 1F. In some examples, the force applied by the rubber band 138 may increase linearly with the amount by which length of the rubber band 138 is stretched from its slack length. In some examples, the force applied by the rubber band 138 may increase in a different manner and/or based on different factors and parameters.

The magnitude of the force applied by the example extension assist mechanism shown in FIG. 1E may be adjustable. For example, the rubber band 138 may be adjusted (e.g., extended or contracted) with respect to the plates 136, the rubber band 138 may be replaced by another rubber band or another type of spring, additional springs may be used, and/or the force may be adjusted based on other modifications.

FIG. 1H shows a side view of an example prosthetic knee 108c. FIG. 1I shows a front view of the example prosthetic knee 108c of FIG. 1H. The example prosthetic knee 108c includes the features of the example prosthetic knee 108a shown in FIGS. 1B, 1C, 1E, 1F, and 1G. The prosthetic knee 108c additionally includes an extension assist mechanism. In some cases, the prosthetic knee 108c may be modified or adapted to include one or more features of the example prosthetic knees shown in FIGS. 1A-1G, 1J, 3A-3B, 4A-4B, 5A-5D, 6A-6C, and 16A-16B, and/or other types of prosthetic knees.

The example extension assist mechanism of the prosthetic knee 108c shown in FIGS. 1H and 1I includes a rubber band 172 that extends the full vertical length of the prosthetic knee 108c. The upper member 120 of the prosthetic knee 108c is coupled to a pyramid adapter 170 carried on the bolt 125, and the lower member 122 of the prosthetic knee 108c is coupled to the pylon 110 secured in the bore 133. The prosthetic knee 108c includes an adapter 171 secured to the anterior of the upper member 120. The adapter 171 may be made of the same material as the upper member 120 (plastic) and/or a different material. The adapter 171 may be secured to the upper member 120 by screws, adhesive and/or other types of fasteners.

The rubber band 172 extends around the pyramid adapter 170 on top of the upper member 120, down the front of the adapter 171 secured to the upper member 120, down the front of the lower member 122, and around the pylon 110. The rubber band 172 may be secured and/or guided by screws, eye bolts, and/or similar devices secured to the lower member 120, the adapter 171, and/or the upper member 120. For example, the eye bolts may prevent the rubber band 172 from slipping off of the anterior face of the prosthetic knee 108c when the prosthetic knee 108c is flexed.

The rubber band 172 provides extension assist in a similar manner as the rubber band 138 of the example prosthetic knee 108b in FIG. 1E. The rubber band 172 has a slack length, and stretching the rubber band 172 beyond its slack length stores energy in the rubber band 172. When the rubber band 172 is stretched from its slack length by the bending motion of the prosthetic knee 108c, the rubber band 172 exerts a force on the upper member 120 and the lower member 122 forcing the prosthetic knee toward the extended position. The rubber band 172 may be at its slack length or stretched beyond its slack length when the prosthetic knee 108c is in the extended position.

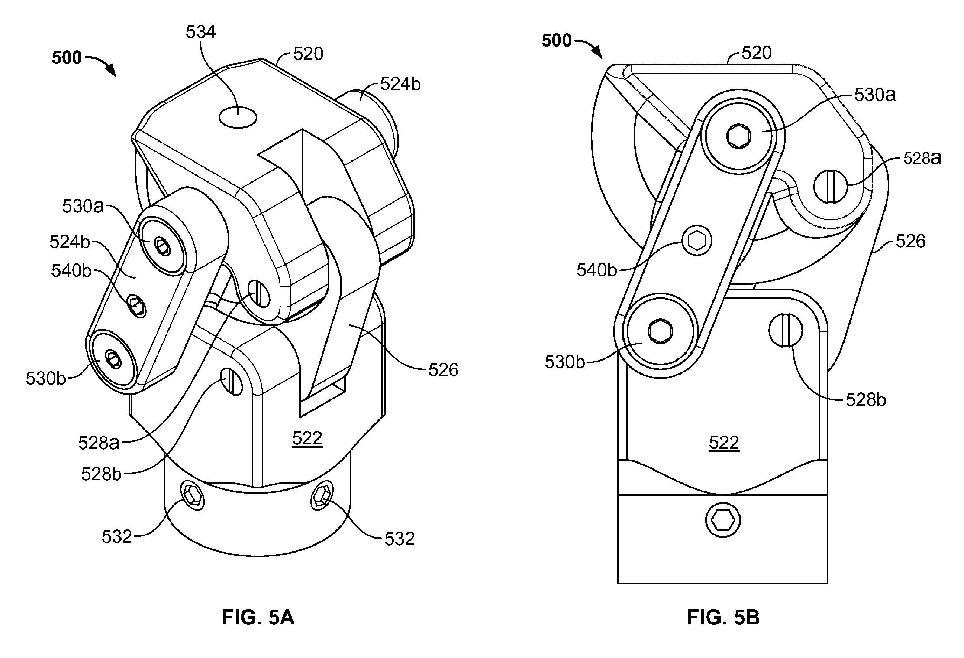

FIG. 1J shows a side view of an example prosthetic knee 108d. The example prosthetic knee 108d includes the features of the example prosthetic knee 108a shown in FIGS. 1B, 1C, 1E, 1F, and 1G. The prosthetic knee 108c additionally includes a locking mechanism. Locking mechanisms can be implemented in a variety of ways using different types of devices. A locking mechanism allows the prosthetic knee to be secured at a fixed angle or in a fixed range of angles of flexation. For example, some locking mechanisms may allow the prosthetic knee to be locked in the extended position, in the flexed position, and/or at one or more intermediate angles of flexation. Example locking mechanisms include pins, wedges, stops, and others. The locking pins of the example prosthetic knee 500 of FIGS. 5A-5D are another type of locking mechanism that can be used. As another example of a locking mechanism, a wedge or block may be inserted between the upper member and the lower member of a prosthetic knee to prevent or restrict flexation of the prosthetic knee. In some cases, the prosthetic knee 108d may be modified or adapted to include one or more features of the example prosthetic knees shown in FIGS. 1A-1I, 3A-3B, 4A-4B, 5A-5D, 6A-6C, and 16A-16B, and/or other types of prosthetic knees.

The example locking mechanism shown in FIG. 1J includes an upper housing 180 secured to the anterior of the upper member 120 and a lower housing 186 secured to the anterior of the lower member 122. The upper housing 180 includes an upper bore 181 that can receive a locking pin 182. The locking pin 182 can be a screw, bolt, rod, pin, and/or another type of mechanism. In the example shown, the locking pin 182 is a threaded fastener coupled to a threaded wing nut 183. The lower housing 186 includes a spring 185 and a lower bore 184. A lower end of the locking pin 182 resides in the lower bore 184 and couples to the spring 185. The spring 185 applies a biasing force to the pin 182, tending to force the pin 182 toward the lower bore 185. The wing nut 183 can be rotated with respect to the pin 182 to adjust the position of the pin 182 with respect to the upper bore 181 and the lower bore 184. The prosthetic knee 108d is locked in the extended position when the pin 182 extends through the upper bore 181 and the lower bore 184. The prosthetic knee 108d is unlocked when the pin 182 has been removed from the upper bore 181 and withdrawn into the lower bore 184.

In the example shown, the links 124a, 124b, 126 each rotate with respect to the upper member 120 and with respect to the lower member 122 during the bending motion of the prosthetic knee 108a. The anterior links 124a, 124b rotate with respect to the upper member 120 on the upper anterior axis (bolt 130a). The anterior links 124a, 124b rotate with respect to the lower member 122 on the lower anterior axis (bolt 130b). The posterior link 126 rotates with respect to the upper member 120 on the upper posterior axis (pin 128a). The posterior link 126 rotates with respect to the lower member 122 on the lower posterior axis (pin 128b).

In the example shown, the lower member 122 rotates with respect to the upper member 120 during the bending motion of the prosthetic knee 108a. The rotation of the lower member 122 with respect to the upper member 120 is a result of the links 124a, 124b, 126 rotating with respect to the upper member 120 and/or with respect to the lower member 122. Rotation of the lower member 122 with respect to the upper member 120 is not a single-axis rotation, but a polycentric rotation wherein an instantaneous center of rotation 131 is an imaginary axis that moves during the bending action of the prosthetic knee 108a.

The location of the instantaneous center of rotation 131 is shown for the prosthetic knee in the extended position in FIG. 1F. The location of the instantaneous center of rotation 131 is shown for the prosthetic knee in the flexed position in FIG. 1G. The instantaneous center of rotation 131 translates between the two positions shown during the bending motion of the prosthetic knee 108a. The example extended position shown corresponds to a bending angle of zero degrees, and the example flexed position shown corresponds to a bending angle of approximately 136 degrees. In some implementation, the flexed position may correspond to a different bending angle.

In the example prosthetic knee 108a, the mechanical axes of rotation, *i.e.,* the upper posterior axis (pin 128a), lower posterior axis (pin 128b), upper anterior axis (bolt 130a), and lower anterior axis (bolt 130b) define the instantaneous center of rotation of the knee. The mechanical axes of rotation are all parallel to each other. As such, each pair of axes defines a plane. Two of the planes intersect at the instantaneous center of rotation. The upper posterior axis (pin 128a) and the lower posterior axis (pin 128b) define a first plane 127 shown in FIGS. 1F and 1G; the upper anterior axis (bolt 130a) and lower anterior axis (bolt 130b) define a second plane 129 shown in FIGS. 1F and 1G. The instantaneous center of rotation 131 of the example prosthetic knee 108a is located at an intersection between a first plane 127 extending through the posterior axes (pins 128a, 128b) and a second plane 129 extending through the anterior axes (bolts 130a, 130b). As such, the instantaneous center of rotation 131 is an imaginary axis parallel to the mechanical axes of rotation and located at the intersection of the first plane 127 and the second plane 129. As the prosthetic knee 108 bends, the mechanical axes of rotation remain parallel to each other, and the upper axes (pin 128a, bolt 130a) move with respect to the lower axes (pin 128b, bolt 130b). As such, the instantaneous center of rotation 131 moves as the prosthetic knee 108a bends from the extended position to the flexed position. In the example shown, when the prosthetic knee 108a is in the extended position as in FIG. 1F, the instantaneous center of rotation 131 is located outside of the prosthetic knee 108a, and when the prosthetic knee 108a is in the flexed position as in FIG. 1G, the instantaneous center of rotation 131 is located inside the prosthetic knee 108a.

In some implementations, the instantaneous center of rotation 131 of the prosthetic knee 108a projected onto a plane perpendicular to the mechanical axes of rotation traces out the path shown in the example plot 200 in FIG. 2. In some implementations, the instantaneous center of rotation of a prosthetic knee projected onto a plane perpendicular to the mechanical axes of rotation traces out a different path. For example, the instantaneous center of rotation may trace out a shorter path, a longer path, a path having a different curvature, a different radius, and/or other features that are different or similar from the example in FIG. 2.

The example plot 200 in FIG. 2 shows the spatial coordinates of the instantaneous center of rotation of the example prosthetic knee 108a and 108b of FIGS. 1B-1G. The plot 200 includes an x-axis in units of millimeters and a y-axis in units of millimeters. The x-axis and y-axis define an xy-plane that is perpendicular to the four mechanical axes of rotation of the prosthetic knees 108a, 108b and in the reference frame of the upper member 120. The upper member 120 is represented in the plot 200 to show the orientation of the upper member 120 with respect to the x- and y-axes, and the upper member 120 is not necessarily drawn to scale in the plot 200. Several points traversed by the instantaneous center of rotation are plotted in the plot 200, and the points are connected by a line 201 showing the approximate path of the instantaneous center of rotation through space.

The instantaneous center of rotation of the prosthetic knee may traverse the path shown by the line 201 in both directions as the knee is flexed or extended. When the prosthetic knee is in the extended position, the center of rotation begins at the highest point 202a, which is located above and posterior to the prosthetic knee. The point 202a may correspond to the location of the instantaneous center of rotation 131 shown in FIG. 1F. As the prosthetic knee begins its bending motion by flexing from the extended position, the instantaneous center of rotation moves toward the second point 202b, which is below and anterior to the first point 202a. The instantaneous center of rotation continues to move as the prosthetic knee flexes further. When the prosthetic knee reaches the fully flexed position, the instantaneous center of rotation terminates at the final point 202c, which may be located within the upper member 120. The point 202c may correspond to the location of the center of rotation 131 shown in FIG. 1G. From the fully flexed position, as the knee extends, the instantaneous center of rotation traces the path shown by the line 201 in the opposite direction, beginning at point 202c and terminating at point 202a.

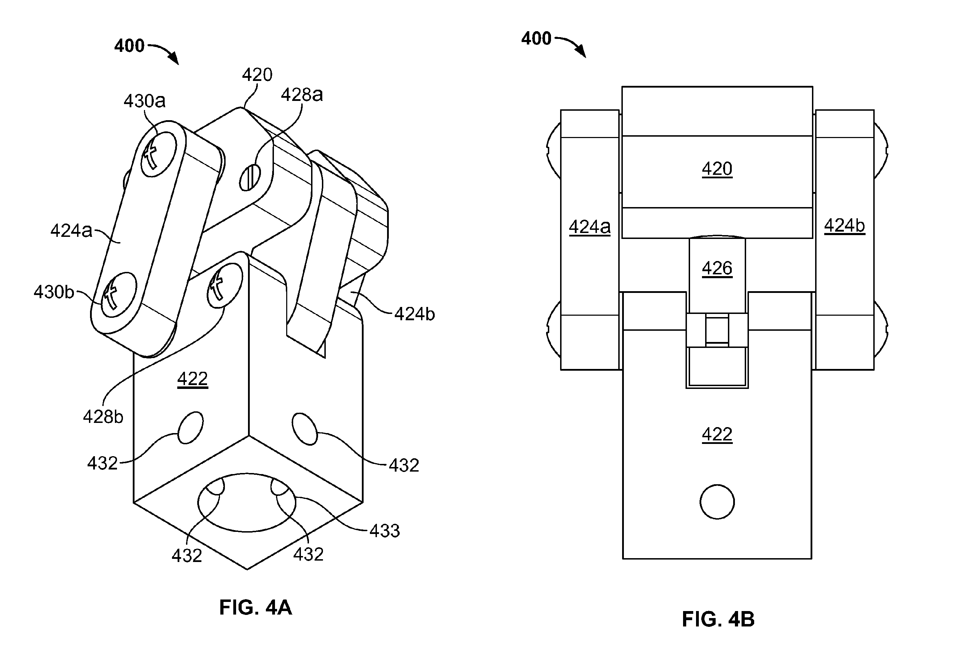

FIG. 4A is a perspective view of an example prosthetic knee 400. FIG. 4B is a front view of the example prosthetic knee 400. In some cases, the prosthetic knee 400 may be modified or adapted to include one or more features of the example prosthetic knees shown in FIGS. 1A-1J, 3A-3B, 5A-5D, 6A-6C, and 16A-16B, and/or other types of prosthetic knees. The prosthetic knee 400 may include an extension assist mechanism, such as the example extension assist mechanism of the knee 108b in FIG. 1E. The example prosthetic knee 400 includes an upper member 420, a lower member 422, two anterior links 424a, 424b, and a posterior link 426. The upper member 420, the lower member 422, the two anterior links 424a, 424b, and the posterior link 426 can be made by hand using cutting tools *(e.g.,* a hack saw, a band saw, or other types of cutting tools), drilling tools *(e.g.,* a hand drill, a drill press, and/or other types of drilling tools), sanding tools *(e.g.,* a sanding belt, a sanding block, and/or other types of sanding tools), and other types of hand tools (e.g., measuring tools, screwdrivers, hammers, wrenches, etc.). Some of the edges and corners of the upper member 420, the lower member 422, the two anterior links 424a, 424b, and the posterior link 426 are rounded. Rounding the edges and corners can make the knee more light weight and may provide a more dynamic profile, smoother surfaces, and a more streamlined appearance.

The example prosthetic knee 400 of FIGS. 4A and 4B includes four axes of mechanical rotation: an upper posterior axis, a lower posterior axis, an upper anterior axis, and a lower anterior axis. In the example shown, the upper posterior axis is formed by a pin 428a, the lower posterior rotational axis is formed by a pin 428b, the upper anterior axis is formed by a bolt 430a, the lower anterior rotational axis is formed by a bolt 430b. The bolts can be secured by nuts and/or other suitable hardware. Additional and/or different types of devices and/or hardware may be used to form the axes of mechanical rotation. For example, any suitable type of bolt, screw, pin, rod, bar, standoff, or similar device may be used to form any of the mechanical axes of rotation. The bolts 430a, 430b include rounded heads, and the bolts 430a, 430b are secured by barrel nuts (not shown). The anterior links 424a, 424b and the posterior link 426 can rotate on the mechanical axes. When the prosthetic knee 400 flexes or extends, the lower member 422 rotates with respect to the upper member 420 about an instantaneous center of rotation defined by the upper posterior axis, the lower posterior axis, the upper anterior axis, and the lower anterior axis.

The lower member 422 of the example prosthetic knee 400 includes a bore 433 that receives an upper end of a pylon. Four fastener bores 432 extend from an outer surface of the lower member 422 to the bore 433. The fastener bores 432 are evenly distributed around the circumference of the bore 433. Two of the fastener bores 432 are parallel to the mechanical axes of rotation (formed by the bolts 430a, 430b and pins 428a, 428b), and two of the fastener bores 432 are perpendicular to the mechanical axes of rotation.

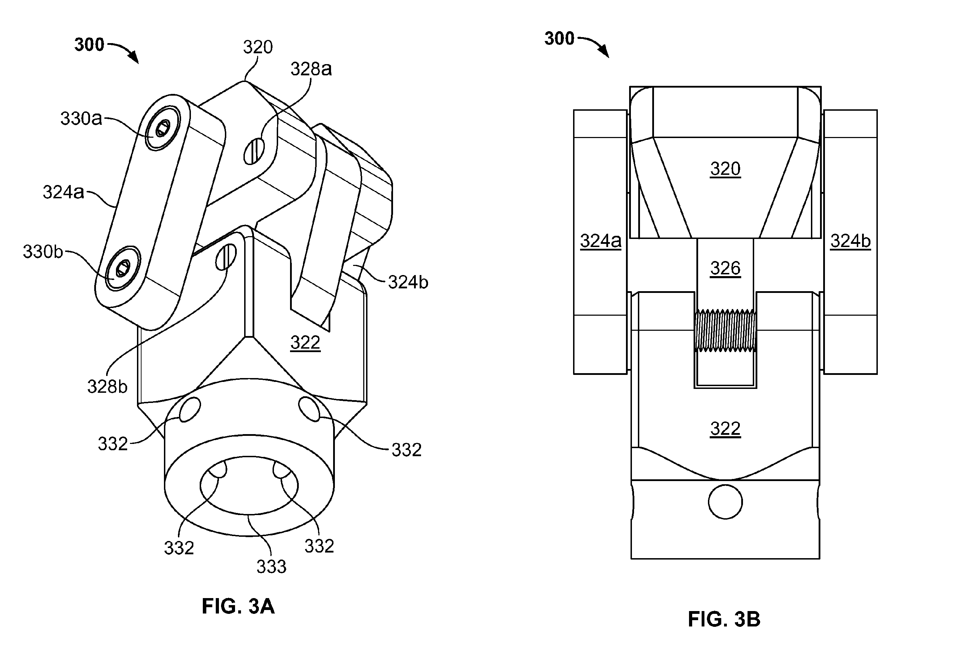

FIG. 3A is a perspective view of an example prosthetic knee 300. FIG. 3B is a front view of the example prosthetic knee 300. In some cases, the prosthetic knee 300 may be modified or adapted to include one or more features of the example prosthetic knees shown in FIGS. 1A-1J, 4A-4B, 5A-5D, 6A-6C, and 16A-16B, and/or other types of prosthetic knees. The prosthetic knee 300 may include an extension assist mechanism, such as the example extension assist mechanism of the knee 108b in FIG. 1E. The example prosthetic knee 300 includes an upper member 320, a lower member 322, two anterior links 324a, 324b, and a posterior link 326. The upper member 320, the lower member 322, the two anterior links 324a, 324b, and the posterior link 326 may be made by a computed numerically controlled (CNC) machining process, an injection pressed molding process, and/or other types of fabrication processes. The machining process may include use of lathes and/or other machining equipment. The lower end of the lower member 322 has a rounded, narrow profile. Some of the edges and corners of the upper member 320, the lower member 322, the two anterior links 324a, 324b, and the posterior link 326 are rounded. Rounding the edges and corners can make the knee more light weight and may provide a more dynamic profile, smoother surfaces, and a more streamlined appearance. The anterior links 324a, 324b include recesses to accommodate countersunk bolts, which may provide a narrower knee profile.

The example prosthetic knee 300 of FIGS. 3A and 3B includes four axes of mechanical rotation: an upper posterior axis, a lower posterior axis, an upper anterior axis, and a lower anterior axis. In the example shown, the upper posterior axis is formed by a pin 328a, the lower posterior rotational axis is formed by a pin 328b, the upper anterior axis is formed by a bolt 330a, the lower anterior rotational axis is formed by a bolt 330b. The bolts can be secured by nuts and/or other suitable hardware. Additional and/or different types of devices and/or hardware may be used to form the axes of mechanical rotation. For example, any suitable type of bolt, screw, pin, rod, bar, standoff, or similar device may be used to form any of the mechanical axes of rotation. The bolts 330a, 330b are countersunk in the recesses in the anterior links 324a, 324b to reduce the profile of the prosthetic knee 300. The heads of the bolts 330a, 330b are countersunk in the anterior link 324a, and the nuts (not shown) that secure the bolts 330a, 330b, are countersunk in the anterior link 324b. The anterior links 324a, 324b and the posterior link 326 can rotate on the mechanical axes. When the prosthetic knee 300 flexes or extends, the lower member 322 rotates with respect to the upper member 320 about an instantaneous center of rotation defined by the upper posterior axis, the lower posterior axis, the upper anterior axis, and the lower anterior axis.

The lower member 322 of the example prosthetic knee 300 includes a bore 333 that receives an upper end of a pylon. Four fastener bores 332 extend from an outer surface of the lower member 322 to the bore 333. The fastener bores 332 are evenly distributed around the circumference of the bore 333. Two of the fastener bores 332 are parallel to the mechanical axes of rotation (formed by the bolts 330a, 330b and pins 328a, 328b), and two of the fastener bores 332 are perpendicular to the mechanical axes of rotation.

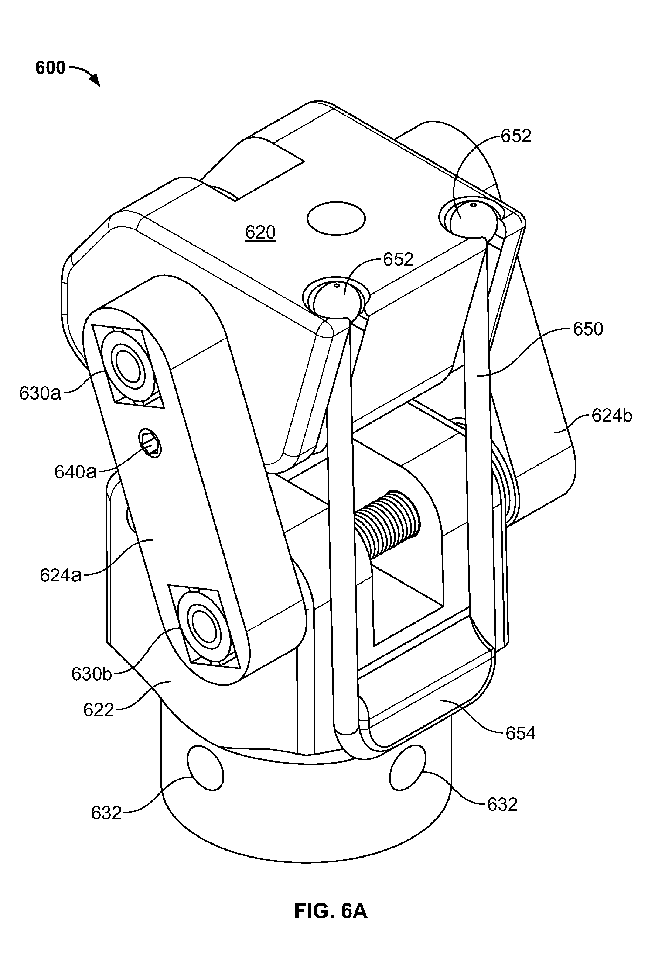

FIG. 6A is a perspective view of an example prosthetic knee 600. Alternative views of the components of the prosthetic knee 600 are shown in FIGS. 6B and 6C. FIG. 6C is a perspective view of the upper member 620 of the example prosthetic knee 600. FIG. 6C is a side view of the lower member 622 of the example prosthetic knee 600 of FIG. 6A. In some cases, the prosthetic knee 600 may be modified or adapted to include one or more features of the example prosthetic knees shown in FIGS. 1A-1J, 3A-3B, 4A-4B, 5A-5D, and 16A-16B, and/or other types of prosthetic knees.

The example prosthetic knee 600 includes an upper member 620, a lower member 622, two anterior links 624a, 624b, and a posterior link (not shown). The upper member 620, the lower member 622, the two anterior links 624a, 624b, and the posterior link 626 can made by a CNC injection technique and/or by another technique. Some of the edges of the upper member 620, the lower member 622, the two anterior links 624a, 624b, and the posterior link 626 are rounded. The upper member 620 includes two grooves 656 that receive the ends of a locking pin 650. The lower member 622 includes a lip 654 that receives a middle portion of the locking pin 650. When the locking pin 650 is applied to the prosthetic knee 600 as shown in FIG. 6A, the prosthetic knee 600 is locked in the extended position. The knobs of the locking pin 650 in the grooves 656 and middle of the locking pin 650 against the lip 654 prevent the upper member 620 and the lower member 622 from rotating with respect to one another. The locking pin 650 may be removed from the prosthetic knee 600, thus allowing the prosthetic knee 600 to flex. The anterior links 624a, 624b include locking pins that may be used to lock the prosthetic knee 600 in an extended position. The locking pin 640a in the anterior link 624a is shown in FIG. 6A.

The example prosthetic knee 600 of FIG. 6 includes four axes of mechanical rotation: an upper posterior axis, a lower posterior axis, an upper anterior axis, and a lower anterior axis. In the example shown, the upper posterior axis is formed by a pin (not shown), the lower posterior rotational axis is formed by a pin (not shown), the upper anterior axis is formed by a bolt 630a, the lower anterior rotational axis is formed by a bolt 630b. The bolts can be secured by nuts and/or other suitable hardware. Additional and/or different types of devices and/or hardware may be used to form the axes of mechanical rotation. For example, any suitable type of bolt, screw, pin, rod, bar, standoff, or similar device may be used to form any of the mechanical axes of rotation. The bolts 630a, 630b are countersunk in the recesses in the anterior links 624a, 624b to reduce the profile of the prosthetic knee 600. When the prosthetic knee 600 is not locked, the anterior links 624a, 624b and the posterior link 626 can rotate on the mechanical axes. When the prosthetic knee 400 flexes or extends, the lower member 622 rotates with respect to the upper member 620 about an instantaneous center of rotation defined by the upper posterior axis, the lower posterior axis, the upper anterior axis, and the lower anterior axis.

As shown in FIG. 6B, the upper member 620 includes an upper bore 634 and at least three lateral bores 642b, 668a, and 670a. The upper member 620 may also include an additional lateral bore 642a opposite the lateral bore 642b. The bores 670a and 668a extend through the width of the upper member 120. The bore 670a carries the bolt 630a that forms the upper anterior axis of the knee 600, and the bore 668a carries the pin that forms the upper posterior axis of the knee 600. Each of the bores 634, 642a, and 642b may terminate at a depth in the upper member 620 or extend through the upper member 620. In some implementations, a bolt or another type of fastener extends through the bore 634 and secures the prosthetic knee 600 to a socket and/or a socket adapter. The bores 642a, 642b can receive the locking pins 640a, 640b, respectively, from the anterior links 624a, 624b. The locking pins 640a, 640b may be inserted in the bores 642a, 642b to lock the prosthetic knee 600, and the locking pins 640a, 640b may be removed from the bores 642a, 642b to unlock the prosthetic knee 600.

As shown in FIG. 6C, the lower member 622 includes at least three lateral bores 632, 668b, and 670b. The lower member 622 may include three additional bores 632 on each of the four faces of the lower member 622. The bores 670b and 668b extend completely through the upper member 620. The bore 670b carries the bolt 630b that forms the lower anterior axis of the knee 600, and the bore 668b carries the pin that forms the lower posterior axis of the knee 600. The four bores 632 may each carry a set screw. The set screws in the bores 632 may extend to an interior of the bore (not shown) to secure a pylon or pylon adapter in the interior bore.

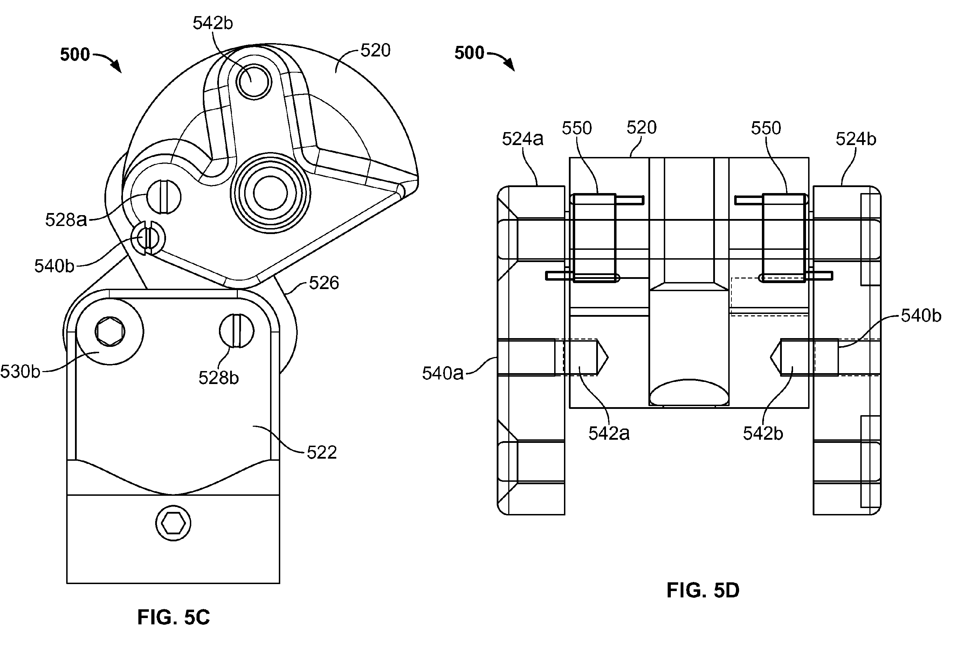

FIG. 5A is a perspective view of an example prosthetic knee 500. Alternative views of the example prosthetic knee 500 are shown in FIGS. 5B, 5C, and 5D. FIG. 5B shows a side view of the example prosthetic knee 500 in an extended position; FIG. 5C shows a side view of the example prosthetic knee 500 in a flexed position; FIG. 5D is a diagram showing internal features of the example prosthetic knee 500. In some cases, the prosthetic knee 500 may be modified or adapted to include one or more features of the example prosthetic knees shown in FIGS. 1A-1J, 3A-3B, 4A-4B, 6A-6C, and 16A-16B, and/or other types of prosthetic knees.

The example prosthetic knee 500 includes an upper member 520, a lower member 522, two anterior links 524a, 524b, and a posterior link 526. The upper member 520, the lower member 522, the two anterior links 524a, 524b, and the posterior link 526 can made by an injection pressed molding technique, and/or by another technique. Some of the edges and corners of the upper member 520, the lower member 522, the two anterior links 524a, 524b, and the posterior link 526 are rounded. Rounding the edges and corners can make the knee more light weight and may provide a more dynamic profile, smoother surfaces, and a more streamlined appearance. The upper member 520 includes a rounded anterior face. The rounded anterior face may emulate the appearance of a human knee cap, providing a more natural appearance.

In the example prosthetic knee 500, as shown in FIG. 5D, the anterior links 524a, 524b each include a locking pin 540a, 540b respectively. The upper member 520 includes locking bores 542a, 542b that received the locking pins 540a, 540b to lock the prosthetic knee 500 in the extended position. The locking pin 540b can be driven from the anterior link 524b into the locking bore 542b and/or the locking pin 540a can be driven from the anterior link 524a into the locking bore 542a to lock the knee in the extended position. The locking pins may be threaded and/or otherwise secured in the anterior links 524a, 524b when the prosthetic knee 500 is not locked. The locking pins may be inserted in the bores to lock the prosthetic knee 500, and the locking pins may be removed from the locking bores to unlock the prosthetic knee 500.

The example prosthetic knee 500 of FIG. 5 includes four axes of mechanical rotation: an upper posterior axis, a lower posterior axis, an upper anterior axis, and a lower anterior axis. In the example shown, the upper posterior axis is formed by a pin 528a, the lower posterior rotational axis is formed by a pin 528b, the upper anterior axis is formed by a bolt 530a, the lower anterior rotational axis is formed by a bolt 530b. The bolts can be secured by nuts and/or other suitable hardware. Additional and/or different types of devices and/or hardware may be used to form the axes of mechanical rotation. For example, any suitable type of bolt, screw, pin, rod, bar, standoff, or similar device may be used to form any of the mechanical axes of rotation. The bolts 530a, 530b are countersunk in the recesses in the anterior links 524a, 524b to reduce the profile of the prosthetic knee 500. When the knee 500 is not locked, the anterior links 524a, 524b and the posterior link 526 can rotate on the mechanical axes. When the prosthetic knee 500 flexes or extends, the lower member 522 rotates with respect to the upper member 520 about an instantaneous center of rotation defined by the upper posterior axis, the lower posterior axis, the upper anterior axis, and the lower anterior axis.

As shown in FIG. 5D, the example prosthetic knee 500 may include an extension assist mechanism. The extension assist mechanism implemented in the prosthetic knee 500 includes two coil springs 550 in the upper member 520. The coil spring 550 is shown in FIG. 7. The two coil springs 550 resides in recesses on either side of the upper member 520. A first end of each coil spring 550 resides in a bore in the upper member 520, and a second end of each coil spring 550 resides in a bore in the adjacent anterior link.

In the example shown, each coil spring 550 has an equilibrium state, and when the coil spring 550 is wound from its equilibrium state, the coil spring 550 tends to contract back to its equilibrium state, which may exert a force. As such, winding the coil spring 550 from its equilibrium state causes the coil spring 550 to store energy. When the prosthetic knee 500 is in the extended position, as shown in FIG. 5B, the coil spring 550 may be at its equilibrium state, so that the coil spring 550 does not apply a force to the prosthetic knee 500 when the prosthetic knee 500 is in the extended position. Alternatively, when the prosthetic knee 500 is in the extended position, as shown in FIG. 5B, the coil spring 550 may be wound from its equilibrium state, so that the coil spring 550 applies a force to the prosthetic knee 500 when the prosthetic knee 500 is in the extended position. The force applied by the coil spring 550 when the prosthetic knee 500 is in the extended position may tend to hold the prosthetic knee 500 in the extended position.

During at least a portion of the bending motion of the prosthetic knee 500, the coil spring 550 is wound from its slack length. When the coil spring 550 begins to wind from its slack length, the coil spring 550 applies a force to the upper member 520 and to the anterior links 524a, 524b. The force applied by the coil springs 550 tends to force the upper member 520 toward the extended position of the prosthetic knee 500.

In the example shown, after the coil spring 550 begins to wind from its equilibrium state, the force applied by the coil spring 550 increases as the prosthetic knee 500 flexes away from the extended position. That is to say, the force applied by the coil spring 550 is at a maximum when the prosthetic knee 500 is fully flexed, for example, as the prosthetic knee 500 in FIG. 5C. In some examples, the force applied by the coil spring 550 may increase linearly and/or in a different manner based on the bending angle of the prosthetic knee 500 and/or different factors and parameters. The magnitude of the force applied by the example extension assist mechanism shown in FIG. 5D may be adjustable. For example, the amount of force applied by the extension assist may be selected based on the spring constant of the spring 550.

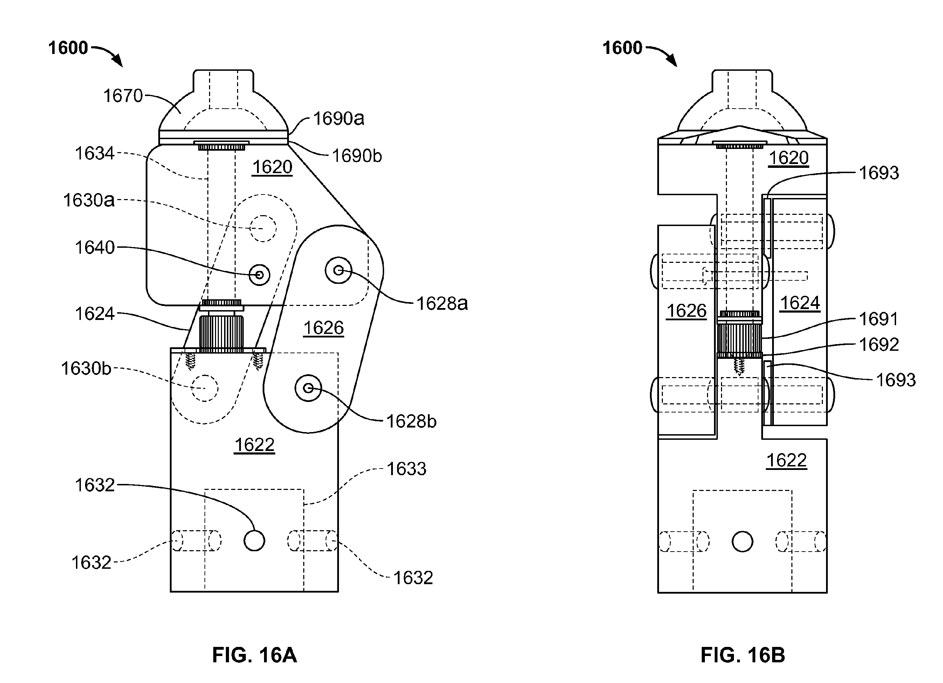

The example polycentric prosthetic knees 108a, 108b, 108c, 108d, 300, 400, 500, and 600 shown and described each include three links that rotatably link the axes of the upper member to the axes of the lower member (two anterior links on the sides of the prosthetic knee, one poster within the sides of the prosthetic knee). In some implementations, a polycentric prosthetic knee may include fewer or more than three links. For example, the example prosthetic knees may be modified to include two or more posterior axes, and/or the example prosthetic knees may be modified to include one, or more than two, anterior axes. An example of a polycentric prosthetic knee 1600 that includes only two links is shown in FIGS. 16A and 16B. By reducing the number of links in the knee, the knee may be made narrower and/or lighter weight. The smaller and/or lighter weight design may be useful for amputees having a small body frame, including small adults and children. In some implementations, mirror images of the example knee 1600 may be used as right and left knees.

FIG. 16A is a side view of the example prosthetic knee 1600 having two links. FIG. 16B is a back view of the example prosthetic knee 1600. In some cases, the prosthetic knee 1600 may be modified or adapted to include one or more features of the example prosthetic knees shown in FIGS. 1A-1J, 3A-3B, 4A-4B, 5A-5D, and 6A-6C, and/or other types of prosthetic knees. The example prosthetic knee 1600 includes an upper member 1620, a lower member 1622, an anterior link 1624, and a posterior link 1626. The upper member 1620, the lower member 1622, the anterior link 1624, and the posterior link 1626 can be manufactured by hand using cutting tools *(e.g.,* a hack saw, a band saw, or other types of cutting tools), drilling tools *(e.g.,* a hand drill, a drill press, and/or other types of drilling tools), sanding tools *(e.g.,* a sanding belt, a sanding block, and/or other types of sanding tools), and other types of hand tools (e.g., measuring tools, screwdrivers, hammers, wrenches, etc.). The upper member 1620, the lower member 1622, the anterior link 1624, and the posterior link 1626 may alternatively be manufactured by different types of manufacturing processes, such as machining and/or molding processes. Edges and corners of the upper member 1620, the lower member 1622, the anterior link 1624, and the posterior link 1626 may be square or rounded.

The example prosthetic knee 1600 of FIGS. 16A and 16B includes four axes of mechanical rotation: an upper posterior axis, a lower posterior axis, an upper anterior axis, and a lower anterior axis. In the example shown, the upper posterior axis is formed by a standoff 628a, the lower posterior rotational axis is formed by a standoff 1628b, the upper anterior axis is formed by a standoff 1630a, and the lower anterior rotational axis is formed by a standoff 1630b. Each of the standoffs 1628a, 1628b, 1630a, 1630b can have a threaded inner bore. Machine screws can be secured in the threaded bore of the standoffs 1628a, 1628b, 1630a, 1630b. Additional and/or different types of devices and/or hardware may be used to form the axes of mechanical rotation. For example, any suitable type of bolt, screw, pin, rod, bar, standoff, or similar device may be used to form any of the mechanical axes of rotation. In the example shown, a spacer 1693 is carried on the machine screw 1630a between the upper member 1620 and the anterior link 1624, and a spacer 1693 is carried on the machine screw 1630b between the lower member 1622 and the anterior link 1626. The spacer may be a washer made of metal, plastic, wood and/or another type of material. The anterior link 1624 and the posterior link 1626 can rotate on the mechanical axes. When the prosthetic knee 1600 flexes or extends, the lower member 1622 rotates with respect to the upper member 1620 about an instantaneous center of rotation defined by the upper posterior axis, the lower posterior axis, the upper anterior axis, and the lower anterior axis.

The example prosthetic knee 1600 includes a rod 1634 extending vertically through the upper member 1620. The rod 1634 can be secured in a bore in the upper member 1620 by captive nuts on the ends of the bore and/or the rod 1634 can be secured in the bore in another manner. The rod 1634 may be threaded or unthreaded. The rod 1634 contacts an adjustable head cap screw 1691 secured to the lower member 1620. The head cap screw 1691 can be adjusted to fine tune the stability of the prosthetic knee 1600. For example, the head cap screw 1691 can be adjusted with respect to the lower member 1622 by rotating the head cap screw 1691. The head cap screw 1691 may also stop flexation of the prosthetic knee 1600. As such, the head cap screw 1691 may be adjusted to fine tune the stop position of the prosthetic knee 1600. An elastomeric pad 1692 may separate the head of the head cap screw 1691 from the lower member 1622. The elastomeric pad 1692 may absorb impact of the rod 1634 contacting the head cap screw 1691 when the prosthetic knee 1600 is extended. The elastomeric pad 1692 may be made of any type of elastomeric material, including rubber, foam, and/or others.

An external load applied to the upper member 1620 may be transferred to the lower member 1622 through the links 1624, 1626 and/or through contact between the rod 1634 and the head cap screw 1691. Thus, the example prosthetic knee 1600 may transfer all or part of an external load from the upper member 1620 to the lower member 1622 fully or partially through the links 1624, 1626; the example prosthetic knee 1600 may transfer all or part of an external load from the upper member 1620 to the lower member 1622 fully or partially through the rod 1634 contacting the head cap screw 1691; or the example prosthetic knee 1600 may transfer an external load from the upper member 1620 to the lower member 1622 through a combination of the links 1624, 1626 and the contact between the rod 1634 and the head cap screw 1691.

The lower member 1622 of the example prosthetic knee 1600 includes a bore 1633 that receives an upper end of a pylon. Four fastener bores 1632 extend from an outer surface of the lower member 1622 to the bore 1633. The fastener bores 1632 are evenly distributed around the circumference of the bore 1633. Two of the fastener bores 1632 are parallel to the mechanical axes of rotation (1628a, 1628b, 1630a, 1630b), and two of the fastener bores 432 are perpendicular to the mechanical axes of rotation.

As shown in FIGS. 16A and 16B, a pyramid adapter 1670 and two wedges 1690a, 1690b are secured to the top of the upper member 1620 of the example prosthetic knee 1600. The pyramid adapter 1670 may allow the prosthetic knee 1600 to couple to one or more types of socket. The wedges 1690a, 1690b may allow angular alignment between the prosthetic knee 1600 and the socket to be adjusted. The wedges 1690a, 1690b may include features of the wedges 1804a, 1804b of FIG. 18. Rotation of either of the wedges 1690a, 1690b with respect to the other wedge and/or rotation of the wedges 1690a, 1690b with respect to the prosthetic knee 1600 and the adapter 1670 may adjust the angular alignment of the prosthetic knee 1600 and the socket. The wedges 1690a, 1690b and/or the pyramid adapter 1670 may be used alone or in combination with other types of adapters and/or similar devices.

The example prosthetic knee 1600 includes a locking mechanism. The locking mechanism includes a locking pin 1640. The locking pin 1640 functions in a manner similar to the locking pins 540a, 540b shown in FIGS. 5A-5D. In the example prosthetic knee 1600, the anterior link 1624 and the upper member 1620 may each include locking pin bores. The locking pin bores may be aligned when the prosthetic knee 1600 is in the extended position. The locking pin 1640 may be secured in the aligned locking pin bores to lock the prosthetic knee in the extended position, which may prevent or reduce flexation of the prosthetic knee. The locking pin 1640 may be removed from the locking pin bores to unlock the prosthetic knee, allowing flexation of the prosthetic knee. The prosthetic knee 1600 may be modified to include additional and/or different types of locking mechanisms.

The example prosthetic knee 1600 may include an extension assist mechanism. For example, the prosthetic knee 1600 may include a rubber band secured to the upper member 1620 and to the lower member 1622 similar to the extension assist mechanism shown in FIG. 1E. As another example, the prosthetic knee 1600 may include a rubber band that extends around the adapter 1670, down the anterior face of the upper member 1620, down the anterior face of the lower member 1622, and around a pylon secured in the bore 1633, similar to the extension assist mechanism shown in FIGS. 1H and 1I. The prosthetic knee 1600 may include additional and/or different types of extension assist mechanisms.

FIG. 8 is a diagram showing an example process 800 for manufacturing a prosthetic knee. As shown in FIG. 8, the example process 800 is used to manufacture the prosthetic knee 108. Aspects of the example process 800 may be used to manufacture the prosthetic knees 108a, 108b, 300, 400, 500, 600, various other prosthetic knees, and/or component parts for a prosthetic knee. FIGS. 8, 9A-9E, 10A-10D, 11, 12A, 12B, 13A-13C, 14, and 15A-15E show example operations, materials, tools, and equipment that may be used in some implementations of the process 800 and/or other manufacturing processes. The operations, materials, tools, and equipment shown in FIGS. 8, 9A-9E, 10A-10D, 11, 12A, 12B, 13A-13C, 14, and 15A-15E may be modified and/or adapted for manufacturing a prosthetic knee in various conditions, using various tools and materials, to produce a prosthetic knee and/or prosthetic knee components having various properties. For example, additional, fewer, or different operations may be performed in the same or a different order to accomplish the same or a different result; additional, fewer, or different materials, tools, and equipment may be used in the same or a different manner to accomplish the same or a different result. In some instances, the operations shown or described can be repeated. In some instances, individual operations and/or subsets of the operations shown or described can be performed in isolation.

In some implementations, the manufacturing process 800 may be used as a low-cost, repeatable, error-tolerant process for manufacturing a polycentric prosthetic knee, such as the prosthetic knees 108, 108a, 108b, and/or other prosthetic knees described herein and their component parts. Aspects of the process 800 may be used to restore, repair, and/or replace components of a previously manufactured prosthetic knee. In some implementations, the process 800 may be implemented by laborers having simple skill sets, using common, inexpensive tools and materials. The example process 800 may utilize parts and materials that can be manufactured and/or purchased in diverse international locations. The process 800 can be adapted for implementation in locations around the world, including many underdeveloped nations.

The example process 800 can be used to manufacture a prosthetic knee 108 from source materials 801 and hardware 810. The source materials 801 may include materials used to fabricate the structural components of the prosthetic knee, including the upper member 120, the lower member 122, the posterior link 126, and the anterior links 124a, 124b. The source material 801 may include raw materials, such as uncut wood, plastic pellets, sheet rubber, and/or other types of raw materials. The source materials 801 may include various sizes of bar stock and round stock materials 802, and/or various sizes of flat stock and sheet stock materials 803a, 803b, 803c. For example, the structural components may be made of solid plastic material that can be easily found, salvaged, and/or purchased in a variety of locations. In the example shown, the upper member 120 and the lower member 122 can be made from 65 mm Diameter Delrin rod and/or 50 mm Delrin sheet source materials, and the anterior links 124a, 124b and the posterior link 126 can be made from 16 mm Delrin sheet source material. Multiple different materials may be used to fabricate the structural components of a prosthetic knee. For example, the upper member and the lower member may be made of a first type of plastic material, and the links may be made of a second type of plastic material. As another example which is not part of the invention, the upper member and the lower member may be made of non-plastic material, and the links may be made of plastic material. As another example, the one or more anterior links may be made of a first type of plastic material, and the one or more posterior links may be made of a second type of plastic material. Dissimilar materials (e.g., multiple types of plastic) may be used in additional and/or different combinations.

Examples of plastic materials that can be used for the upper member, the lower member, the posterior link, the anterior links, and/or other structural components include homopolymer polyacetal plastics, copolymer polyacetal plastics, other materials having similar properties, and combinations thereof. Example homopolymer polyacetal plastics include various types of Delrin (developed by DuPont). Example copolymer polyacetal plastics include various types of Acetron (developed by DuPont). Additional examples of plastic materials that can be used for the upper member, the lower member, the posterior link, the anterior links, and/or other structural components include various types of nylon, acrylic, and others. Additional examples that can be used for one or more embodiments include nylon 6, nylon 66, polyetheretherketone (PEEK), polyetherketone (PEK), polyetherketoneketone (PEKK), polyethylene terephthalate (PET), polyimide (PI), polyarylamide, polyester liquid crystal (liquid crystal polymer, LCP), polyphthalamide (PPA), polyphenylene sulfide (PPS).

The material of the plastic structural components may be selected based on a number of factors. For example, the plastic material may be selected based on availability, price, mechanical properties, cosmetics, compatibility with tools and other devices, and/or other factors. In some instances, the plastic material may include medical grade and/or flesh-colored material.

These and/or other plastic materials may be selected based on their density, ultimate tensile strength, elastic modulus, shear strength, coefficient of friction, coefficient of thermal expansion, melting point, and/or other properties. In some implementations, the upper member, the lower member, the posterior link(s), the anterior link(s), and/or other structural components are made from plastic materials having one or more of the following properties: density less than or equal to 2768 kilogram per cubic meter (kg/m³) [0.1 pounds per cubic inch (lb/in³)], ultimate tensile strength greater than or equal to 655 bar [9,500 pounds per square inch (psi)], elastic modulus greater than or equal to 27,579 bar [400 kilopounds per square inch (ksi)], shear strength greater than or equal to 551.58 bar [8,000 psi], coefficient of friction less than or equal to 0.3, coefficient of thermal expansion less than or equal to 99 micrometer per meter degree Celsius difference [55 microinches per inch - degree Fahrenheit (µin/in·°F)], melting point greater than or equal to 148.9°C [300 degrees Fahrenheit (°F)]. Plastic materials meeting one or more of these example criteria may be used. Additional and/or different materials not meeting these example criteria may be used.

The hardware 810 includes the bolts 130a, 130b, the pins 128a, 128b, washers 820, the nuts 123a, 123b, and screws 822, 132. The bolts 130a, 130b can include M8 x 1.25 x 100 mm bolts and/or other types of bolts. The washers 820 can include M8 washers and/or other types of washers The nuts 123a, 123b can include M8 nylon lock nuts and/or other types of nuts. The screws 822 can include 2.9 mm self-tapping screws and/or other types of screws. The screws 132 can include M10 x 1.5 x 10 mm set screws and/or other types of screws. The pins 128a, 128b can include modified M8 x 1.25 x 60 mm bolts and/or other types of pins. For example, one of the pins 128a, 128b can be made by cutting an M8 x 1.25 x 60 mm bolt to a length of approximately 50 mm; a groove can be formed in the head of the shortened bolt to allow the bolt to be driven by a flat head screwdriver or similar device. Bolts, pins, washers, nuts, screws, and additional or different hardware of other sizes and types may be used. In some cases the bolts, pins, washers, nuts, screws, and/or other hardware are made of one or more types of metal (e.g., steel, copper, iron, aluminum, brass, and/or others), plastic *(e.g.,* nylon, delrin, acetron, and/or others), wood, and/or other materials. Different types of fasteners and support hardware may be used. For example, dowels, pins, rods, bolts, standoffs, and/or similar hardware may be substituted for the bolts 130a, 130b and/or pins 128a, 128b. A standoff may include a threaded inner bore and a non-threaded outer surface; the outer surface may be rounded, faceted, and/or have another type of geometry. As another example, various types and sizes of fasteners may be substituted for the screws 822, 132 and/or the nuts 123a, 123b. In some implementations, the hardware 810 can be found at hardware stores, or may be salvaged or purchased in a variety of locations.

Additional materials and hardware may be used when a locking mechanism and/or an extension assist mechanism is incorporated in the prosthetic knee. For example, springs, rubber bands, and/or other materials and hardware may be used to manufacture a prosthetic knee that includes the example extension assist mechanisms shown in FIGS. 1E and 5E. As another example, locking pins, rods and/or other materials and hardware may be used to manufacture a prosthetic knee that includes the example locking mechanisms shown in FIGS. 5E and 6A.

The process 800 can be implemented using a variety of different tools. Cutting tools and drilling tools may be used to fabricate the components 805 from the source materials 801. Cutting tools may include a hacksaw, a bandsaw, scissors, a utility knife, and/or others. Drilling tools may include a handheld drill, a drill press, a tap, a tap handle, and/or others. Specific drilling apparatus that may be used include a 2 mm drill bit, a 6.5 mm drill bit, an 8 mm drill bit, a 10 mm drill bit, a 17 mm drill bit, a 30 mm forstner bit, a 10mm x 1.5 tap, an 8 mm x 1.25 tap, a 17 mm drill stop, an 8 mm drill stop, and/or others. Sanding tools may also be used to finish the components 805. For example, sanding tools may be used to accomplish the rounded edges of the upper member, the lower member, and the links of the example knees 300, 400, 500, 600. Sanding tools may include sand paper, a sanding block, a sanding belt, a file, and/or others. Other tools that may be used to fabricate the components 805 include a marker, a hammer, a punch, a square, a clamp, a band saw fence, a drill press vice, and/or others. Fastening tools may be used to assemble the components 805 and the hardware 810. Fastening tools may include a screwdriver, a hand drill, a hammer, a wrench, a key, and/or others. Specific types of fastening tools may include a 13 mm wrench, a 5 mm Allen key, a Phillips head screwdriver, a flathead screwdriver, a Phillips head drill bit, a flathead drill bit, and/or others. The process 800 may also use drilling jigs, templates, and/or other models for manufacturing the prosthetic knee 108.

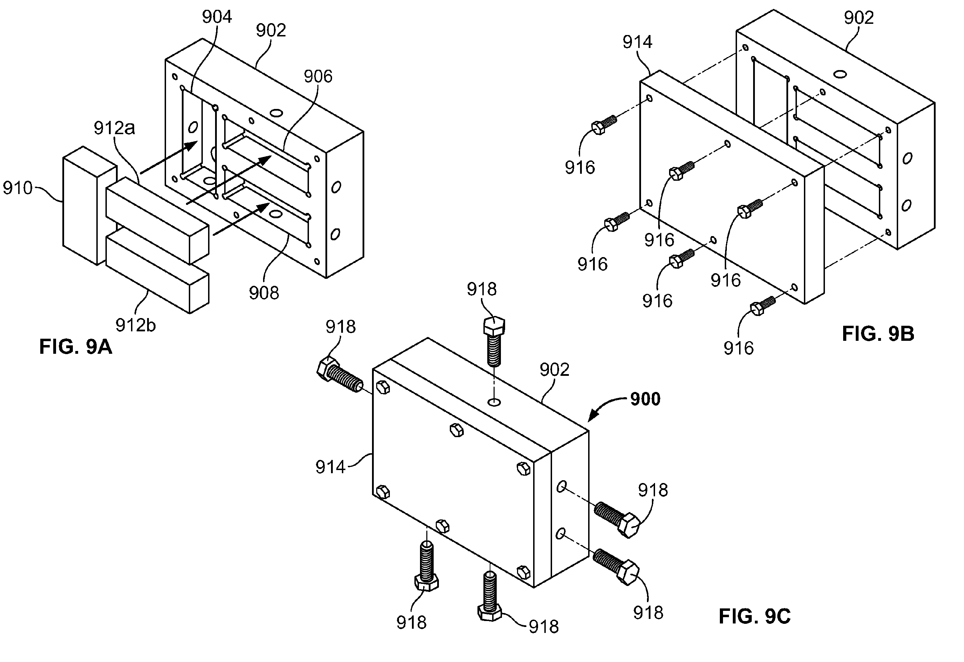

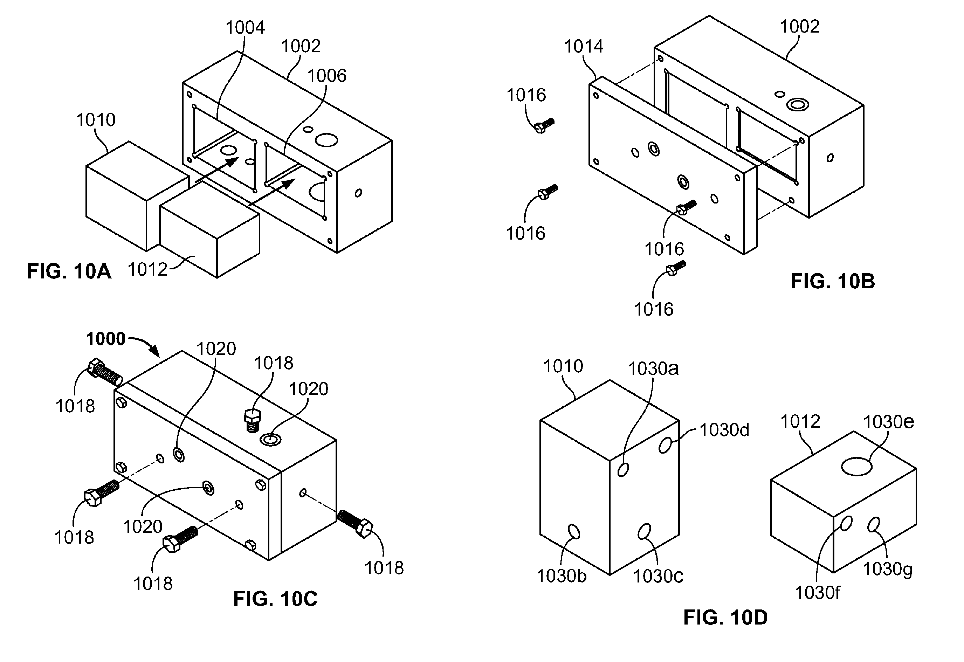

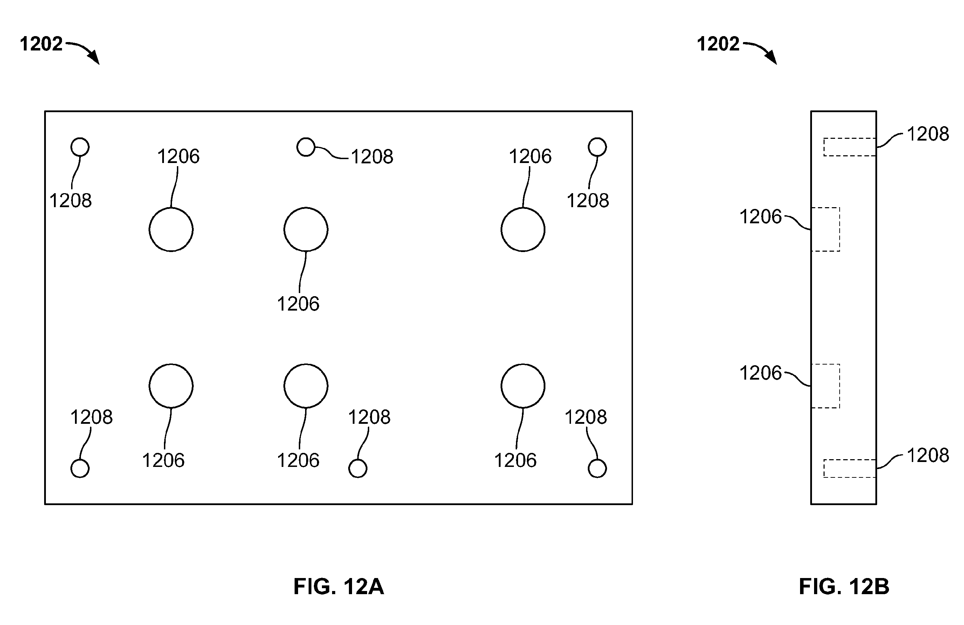

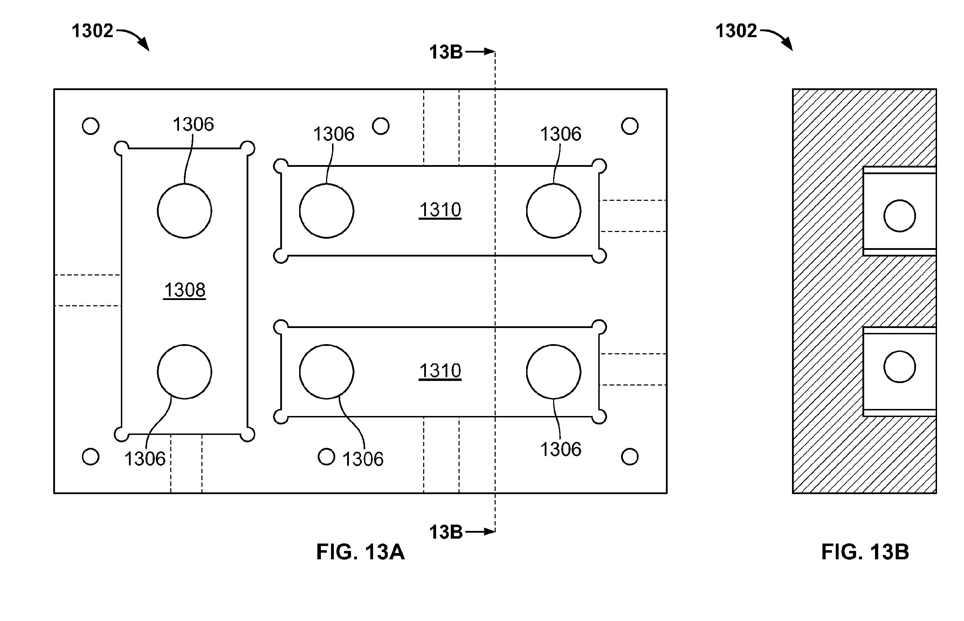

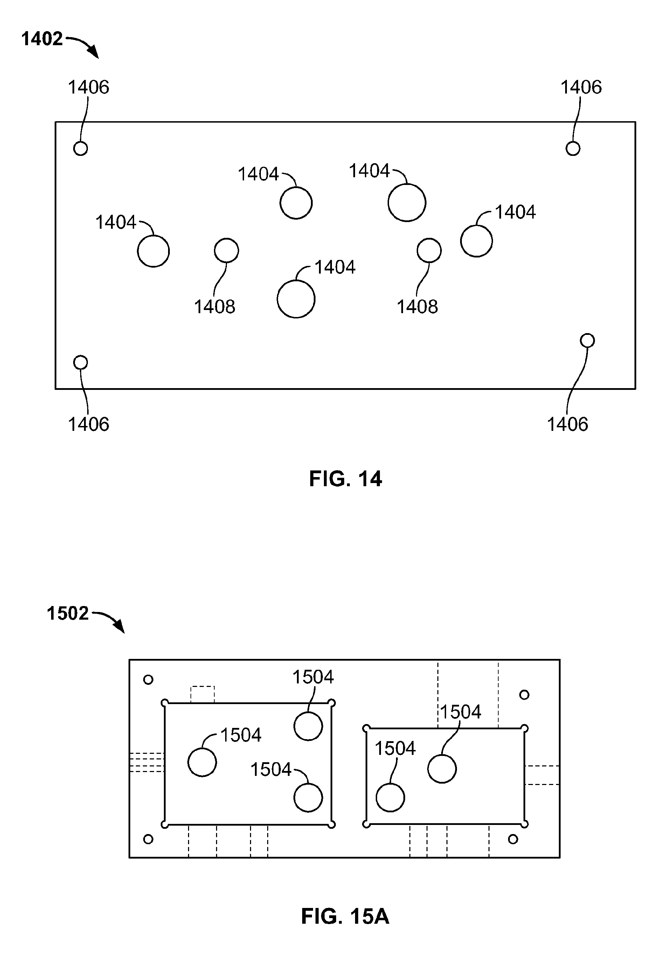

At 804, the structural components 805 are fabricated from the source materials 801. The fabricated structural components 805 include the upper member 120, the lower member 122, the posterior link 126, and the anterior links 124a, 124b. The components 805 may also include a stop 135 (not shown in FIG. 8), which may be cut from a sheet of elastomeric material, for example, using scissors, a utility knife, or another type of cutting device. A stop may be made of any type of elastomeric material, which may include rubber, foam, and/or others. In some implementations, drilling jigs are used in fabricating the components 805. For example, the tools, equipment, and techniques shown in FIGS. 9A-9E may be used in fabricating the links 126, 124a, 124b, and the tools, equipment, and techniques shown in FIGS. 10A-10D may be used in fabricating the upper member 120 and the lower member 122. The drilling jigs shown in FIGS. 12A, 12B, and 13A-13C may be used to fabricate the links 126, 124a, 124b, and the drilling jigs shown in FIGS. 14 and 15A-15H may be used to fabricate the upper member 120 and the lower member 122. In some implementations, templates are used in fabricating the components 805. For example, the components 805 may be fabricated using templates and/or other types of models rather than jigs; or the components 805 may be fabricated using a combination of templates, jigs, and/or other types of models. The templates shown in FIG. 11 may be used in fabricating one or more of the upper member 120, the lower member 122, the posterior link 126, and the anterior links 124a, 124b. The components 805 shown in FIG. 8 are example components. Additional, different, and/or fewer components may be fabricated in various implementations of the process 800.

The components 805 are designed to function properly when they are manufactured within tolerances. The design of the components 805 may allow one or more of the components 805 to be manufactured within tolerances using relatively simple tools (e.g., saw, drill, etc.). For example, the design may include tolerances of approximately one to four or more millimeters. In some example prosthetic knee designs, the length, width, and height of the knee components 805 have a tolerance of up to ±2 mm, the placement of the bores for the mechanical axes has a tolerance of up to ±2 mm, and/or the runout of the bores for the mechanical axes has a tolerance of up to ±2 mm. The runout of the bores relates to the degree to which the bores are parallel. For example, the bores are within tolerance when the distance between them is within the placement tolerance and when they are drilled within the runout tolerance of parallel.

The process 800 and/or operations of the process 800 may be repeated to fabricate multiple interchangeable components 805. For example, an amputee may be fitted with a prosthetic knee and provided one or more spare components 805 that can be installed in the event that a component of the prosthetic knee 108 fails or wears out. By fabricating multiple interchangeable components, individual parts of the knee can be replaced, rather than having to manufacture an entire prosthetic knee every time a component wears out.

At 826, the prosthetic knee 108 is assembled from the components 805 and the hardware 810. The bolt 130a may form the upper anterior axis of the prosthetic knee 180. For example, the bolt 130a may carry four of the washers 820 and the nut 123a and may secure the anterior links 124a, 124b to the upper member 120 as shown in FIGS. 1B-1G. The bolt 130b may form the lower anterior axis of the prosthetic knee 180. For example, the bolt 130b may carry four of the washers 820 and the nut 123b and may secure the anterior links 124a, 124b to the lower member 122 as shown in FIGS. 1B-1G. The bolts 130a, 130b may be tightened, for example, by hand, using a wrench, and/or in another manner. The pin 128a may form the upper posterior axis of the prosthetic knee 108, and the pin 128b may form the lower posterior axis of the prosthetic knee 108. For example, the pin 128a may carry two of the washers 820 and may secure the posterior link 126 to the upper member 120, and the pin 128b may carry two of the washers 820 and may secure the posterior link 126 to the lower member 122 as shown in FIGS. 1B-1G. The pins 128a, 128b may be driven, for example, by hand, using a flat head screwdriver, and/or in another manner. The set screws 132 can be installed in the lower member 122 as shown in FIGS. 1B-1G. The set screws 132 may be driven, for example, using an Allen key. The screw 822 can secure a rubber stop 135 to the upper member 120 as shown in FIG. 1D. A hole for the screw 820 can be driven using a tap, and the screw 820 can be driven by a screwdriver.

Additional assembly may be necessary when a locking mechanism and/or an extension assist mechanism is incorporated in the prosthetic knee. For example, springs, rubber bands, locking pins, and/or other materials and hardware may be installed to implement the locking mechanisms or the extension assist mechanisms shown in FIGS. 1E, 5E, and 6A.

FIGS. 9A-9E are diagrams showing an example technique for using a drilling jig 900 to fabricate links of a prosthetic knee. For example, the drilling jig 900 may be used to fabricate the anterior links 124a, 124b and/or the posterior link 126 shown in FIGS. 1B-1G. The drilling jig 900 can include the cover component 1202 shown in FIGS 12A and 12B, the slot component 1302 shown in FIGS. 13A-13C, and/or other drilling jig components. For example, the drilling jig 900 and/or other drilling jigs may be used to fabricate posterior and/or anterior links having the same or different shapes, sizes, dimensions, and/or geometries.

Drilling jigs may be used to manufacture polycentric prosthetic knees in regions of the world where expensive manufacturing techniques are impractical or unavailable. Drilling jigs may be used to assure precise, accurate placement and orientation of bores in the structural components of a prosthetic knee. In some implementations, the functionality of the prosthetic knee depends on precise spatial relationships among the mechanical axes of rotation and/or other features of the prosthetic knee. For example, in some cases, the mechanical axes of rotation must be nearly exactly parallel, and the distances and angles among the mechanical axes must be placed with great precision. Because the bores in the structural components determine the positions of the bolts, pins, rods, standoffs, and/or other hardware forming the mechanical axes of rotation, precise and accurate placement of the bores may be critical to the functionality of the prosthetic knee. Drilling jigs and simple drilling tools (such as a hand drill, a tap, and/or others) can be used to achieve the requisite precision and accuracy. As such, the drilling jigs may enable technicians with simple skill sets to manufacture prosthetic knees without use of computer guided machining processes, CNC compression molding processes, or other advanced manufacturing techniques. Drilling jigs may be manufactured for durability, to allow hundreds or even thousands of prosthetic knee components to be reliably fabricated using the same drilling jig. As such, a set of drilling jigs may be deployed in a single location and used by local technicians to manufacture and/or repair hundreds or thousands of prosthetic knees.

As shown in FIG. 9A, a posterior link block 910 and two anterior link blocks 912a, 912b are inserted into the slot component 902 of the example drilling jig 900. The slot component 902 includes three slots. The posterior link block 910 is inserted in a posterior link slot 904, and the anterior link blocks 912a, 912b are inserted into the anterior link slots

rubber bands, and/or other materials and hardware may be used to manufacture a prosthetic knee that includes the example extension assist mechanisms shown in FIGS. 1E and 5E. 906 and 908. The posterior link block 910 and the anterior link blocks 912a, 912b can be cut from the source material 801 and sized to fit in the respective slots of the slot component 902.

As shown in FIG. 9B, a cover component 914 is secured to the slot component 902 by six bolts 916. A different number of bolts 916 may be used to secure the cover component 914 to the slot component 902. The bolts 916 can include M4 x 0.7 x 16mm hex bolts and/or a other types of bolts. In some implementations, the cover component 914 is secured to the slot component 902 in a different manner. For example, in addition or as an alternative to the bolts 916, the cover component 914 may be secured to the slot component 902 by a latch, threading, magnets, and/or other types of fasteners.

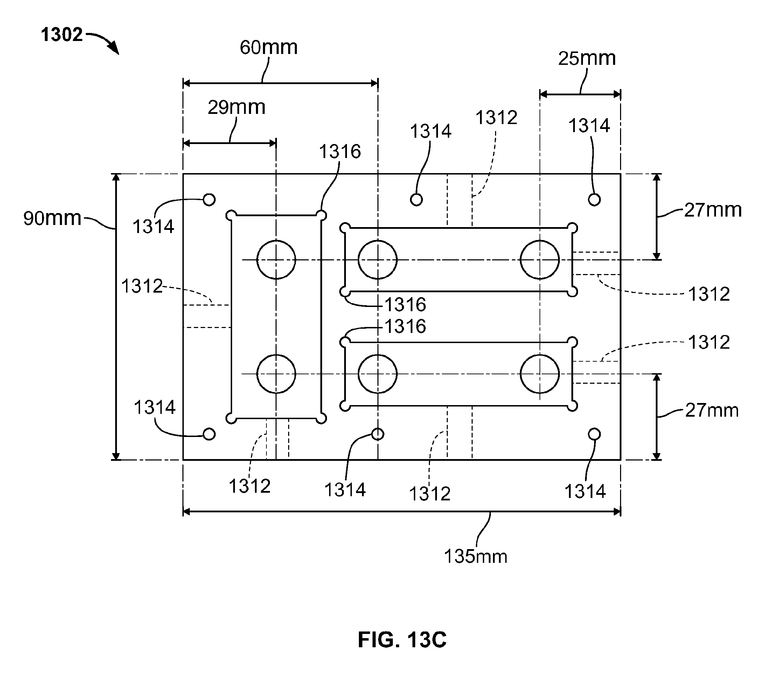

As shown in FIG. 9C, six bolts 918 secure the posterior link block 910 and the two anterior link blocks 912a, 912b in a fixed position in their respective slots 904, 906, 908. A different number of bolts 918 may be used. The bolts 918 may include 8 mm pressure bolts and/or other types of bolts. In the example shown, two of the bolts 918 contact each of the link blocks in the drilling jig 900. The two bolts 918 that contact each link block apply pressure to the link block, forcing the link block to a reference corner of the slot in which it resides. FIG. 13C shows reference corners 1316 in an example drilling jig. Forcing each link bock to the reference corner in the slot assures that the bores are drilled at a specified distance from a specific corner of the link block. Thus, the dimensions of the link blocks 910, 912a, 912b can tolerate some reasonable amount of error, and the link blocks do not have to fit perfectly in the slots 904, 906, 908 to assure precision drilling. Accordingly, the drilling jigs allow the link blocks to be cut from the source materials using simple cutting tools, such as a band saw, a hand saw, or similar cutting equipment. As such, the link blocks 910, 912a, 912b can be fabricated without computer guided machining processes, CNC compression molding processes, or other advanced manufacturing techniques.

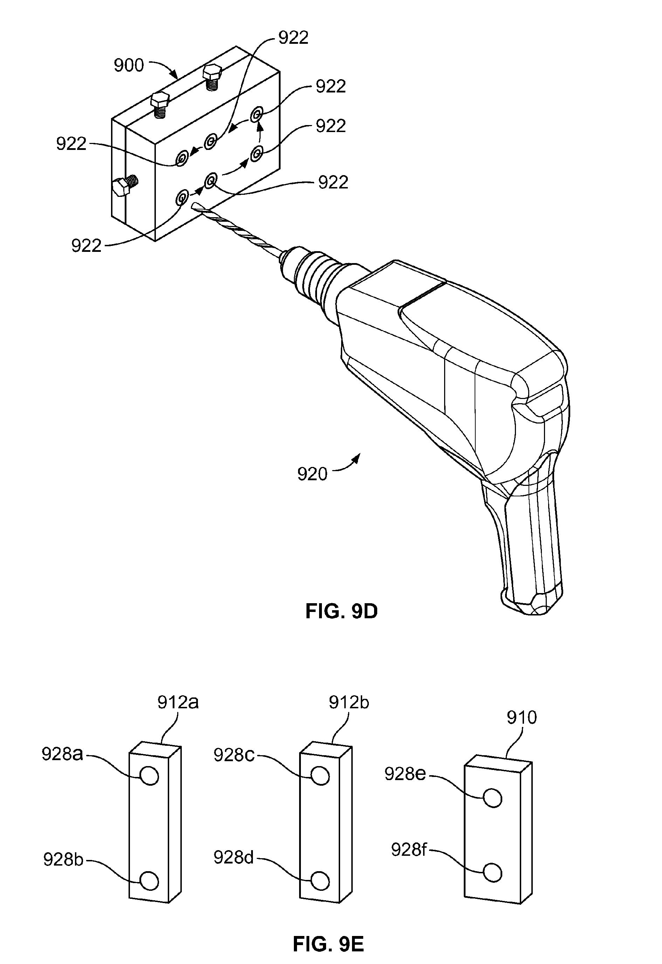

As shown in FIGS. 9D and 9E, a hand drill 920 can be used to drill bores in the link blocks. The example drilling jig 900 includes six drill sites 922. The hand drill 920 can be outfitted with appropriately sized drill bits, an appropriately positioned drill stop, and/or other appropriate drill settings. In some implementations, the bores of the anterior links are drilled with an 8mm drill bit. In some implementations, one or more additional or different sized drill bits are used. The drill sites 922 may be drilled in a preset order to minimize the effects of slippage due to torque applied to the link blocks by the drill bit. In the example shown, the six drill sites 922 are drilled in a counterclockwise order. In some implementations, the order of drilling the drill sites 922 has a minimal or negligible effect. The drill sites 922 may be drilled through the full depth of the slots 904, 906, 908. In some implementations, one or more of the drill sites is drilled to a specified depth, which may be controlled by a drill stop or similar device on the drill 920.

The drill 920 can be any handheld drill with sufficient power to drill the material in the slots of the drilling jig. The drill can be battery powered, powered by anAC voltage source, manually powered, air pressure powered, or powered in a different manner. When the link blocks are made of plastic, the drill 920 can be a standard drill type available from manufacturers such as DeWalt, Ryobi, Black & Decker, and/or others. In some implementations, a drill press, a tap, and/or other types of drilling or cutting tools may be used to drill one or more of the drill sites 922.

Each drill site 922 may include a bushing that reduces wear on the drilling jig 900. For example, the bushings may be made of a more durable material than other parts of the drilling jig 900. The bushings may be made of any material that is harder than the drill bit, for example, hardened steel and/or other materials. The bushings may allow the drill bit to contact the drill sites 922 without modifying (e.g., widening) the drill sites 922. Thus, the bushings allow the drilling jig 900 to produce a predictable, reliable, precise output over many repeated uses. In some implementations, the bushings can withstand hundreds or thousands of uses before needing replacement.

As shown in FIG. 9E, after the drill sites 922 of the drilling jig 900 have been drilled, the link blocks 910, 912a, 912b are removed from the drilling jig 900. As a result of the drilling in FIG. 9D, each of the three link blocks 910, 912a, 912b includes two bores, corresponding to the six drill sites 922 in the drilling jig 900. The anterior link block 912a includes bores 928a, 928b, the anterior link block 912b includes bores 928c, 928d, and the posterior link block 910 includes bores 928e, 928f. The drilled link blocks 910, 912a, 912b shown in FIG. 9E may be further modified to form the links 126, 124a, 124b of FIG. 8. For example, one or more of the templates shown in FIG. 11 may be used to convert the drilled link blocks 910, 912a, 912b shown in FIG. 9E to links 126, 124a, 124b that are used in the prosthetic knee 108.

FIG. 11 is a diagram showing example templates for fabricating components of a prosthetic knee. FIG. 11 shows seven example templates that may be used to fabricate one or more of the components 805 of FIG. 8. For example, the templates may be used to manufacture one or more components of the knee 108a shown in FIGS. 1B-1G and/or other knees. The example templates shown include an anterior link guide 1102, a posterior link guide 1104, bottom block guides 1106, 1108, 1114, and top block guides 1110, 1112. Additional, different, or fewer templates may be used to fabricate the components 805. The templates can be used in manufacturing processes that also utilize drilling jigs, or the templates can be used in manufacturing processes that do not utilize drilling jigs. For example, the holes in the templates may be used as a guide for drilling bores in the components, or the holes in the templates may be used to align the templates with bores drilled with a drilling jig or another type of drilling process.

In some implementations, the posterior link guide 1102 may be used as a template for finishing the posterior link 126. In an example implementation, the holes of the posterior link guide 1102 are aligned with the bores of the posterior link block 910 *(e.g.,* by inserting bolts through the holes and bores), a permanent marker or another writing tool is used to outline the edges of the posterior link guide 1102 on the posterior link block 910, and the sharpie outline is used as a guide for sanding the corners and edges of the posterior link block 910 to form the posterior link 126. The anterior link guide 1104 may be used in a similar manner to convert the drilled link blocks 912a, 912b to finished anterior links 124a, 124b. The posterior link guide 1102 and/or the anterior link guide 1104 may be used in a different manner. In some implementations, the drilled link blocks 910, 912a, 912b are converted to finished links according to other techniques, without use of the templates of FIG. 11.

FIGS. 10A-10D are diagrams showing an example technique for using a drilling jig 1000 to fabricate components of a prosthetic knee. For example, the drilling jig 1000 may be used to fabricate the upper member 120 and/or the lower member 122 shown in FIGS. 1B-1G. The drilling jig 1000 can include the cover component 1402 shown in FIG. 14, the slot component 1502 shown in FIGS. 15A-15H, and/or other drilling jig components. For example, the drilling jig 1000 and/or other drilling jigs may be used to fabricate upper components and/or lower components having the same or different shapes, sizes, dimensions, and/or geometries.

As shown in FIG. 10A, a lower member block 1010 and an upper member block 1012 are inserted into the slot component 1002 of the example drilling jig 1000. The slot component 1002 includes two slots. The lower member block 101 is inserted in a lower block slot 1004, and the upper member block 1012 is inserted into the upper block slot 1012. The lower member block 1010 and the upper member block 1012 can be cut from the source material 801 and sized to fit in the respective slots of the slot component 1002.

As shown in FIG. 10B, a cover component 1014 is secured to the slot component 1002 by six bolts 1016. A different number of bolts 1016 may be used to secure the cover component 1014 to the slot component 1002. The bolts 1016 can include M4 x 0.7 x 16mm hex bolts and/or a other types of bolts. In some implementations, the cover component 1014 is secured to the slot component 1002 in a different manner (e.g., using a latch, threading, magnets, and/or other types of fasteners).

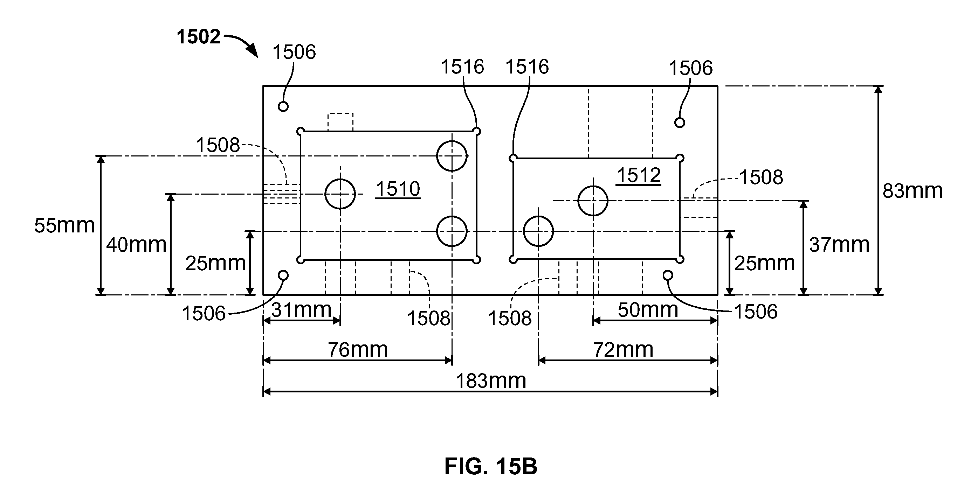

As shown in FIG. 10C, six bolts 1018 secure the lower member block 1010 and the upper member block 1012 in a fixed position in their respective slots 1004, 1006. A different number of bolts 1018 may be used. The bolts 1018 may include 8 mm pressure bolts and/or other types of bolts. In the example shown, three of the bolts 1018 contact each of the blocks in the drilling jig 1000. The three bolts 1018 that contact each link block apply pressure to the link block, forcing the link block to a reference corner of the slot in which it resides. FIG. 15B shows reference corners 1516 in an example drilling jig. Forcing both blocks to the reference corner in the slot assures that the bores are drilled at a specified distance from a specific corner of the block. Thus, the dimensions of the blocks 1010, 1012 can tolerate some reasonable amount of error, and the blocks do not have to fit perfectly in the slots 1004, 1006 to assure precision drilling. Accordingly, the drilling jigs allow the upper and lower member blocks to be cut from the source materials using simple cutting tools, such as a band saw, a hand saw, or similar cutting equipment. As such, the link blocks 1010, 1012 can be fabricated without computer guided machining processes, CNC compression molding processes, or other advanced manufacturing techniques.

A hand drill 920 can be used to drill bores in the lower member block 1010 and the upper member block 1012 in the drilling jig 1000, similar to the drilling shown in FIG. 9D. The example drilling jig 1000 includes several drill sites 1020. The hand drill 1020 can be outfitted with appropriately sized drill bits, an appropriately positioned drill stop, and/or other appropriate drill settings. In some implementations, the bores of the upper member and lower member are drilled using a 6.5 mm drill bit, an 8 mm drill bit, a 10 mm drill bit, a 17 mm drill bit, and a punch and hammer. In some implementations, additional or different sized drill bits are used. The drill sites 1020 may be drilled in a preset order to minimize the effects of slippage due to torque applied to the upper and lower member blocks by the drill bit. In some implementations, the order of drilling the drill sites 1020 has a minimal or negligible effect. The drill sites 1020 may be drilled through the full dimension of the slots 1004, 1006. In some implementations, one or more of the drill sites is drilled to a specified depth, which may be controlled by a drill stop or similar device on the drill 920. Each drill site 1020 may include a bushing that reduces wear on the drilling jig 1000. In some implementations, the bushings can withstand hundreds or thousands of uses before needing replacement.

As shown in FIG. 10D, after the drill sites 1020 of the drilling jig 1000 have been drilled, the lower member block 1010 and the upper member block 1012 are removed from the drilling jig 1000. As a result of the drilling, the lower member block 1010 and the upper member block 1012 include multiple bores, corresponding to the drill sites 1020 in the drilling jig 1000. The lower member block 1010 includes bores 1030a, 1030b, 1030c, 1030d, and possibly other bores. The upper member block 1012 includes bores 1030e, 1030f, and possibly other bores. The bores 1030b and 1030c and two other bores on the opposing faces of the lower member block 1010 may carry the set screws 132, which may be used to secure a pylon 110 to the lower knee member 122. The bore 1030g in the upper member block 1012 may be aligned with the bores 928a, 928c of the anterior links to receive the bolt 130a. The bore 1030d in the lower member block 1010 may be aligned with the bores 928b, 92 8d of the anterior links to receive the bolt 130b. The bore 1030f of the upper member block 1012 may be aligned with the bore 928e of the posterior link to receive the pin 128a. The bore 1030a of the lower member block 1010 may be aligned with the bore 92 8f of the posterior link to receive the pin 128b.

The drilled blocks 1010, 1012 shown in FIG. 10D may be further modified to form the upper member 120 and the lower member 122 of FIG. 8. For example, one or more of the templates shown in FIG. 11 may be used to convert the drilled blocks 1010, 1012 shown in FIG. 10D to the upper member 120 and the lower member 122 that are used in the prosthetic knee 108. In some implementations, the top block guides 1110, 1112 can be used to convert the drilled upper member block 1012 to the upper member 120, and the bottom block guides 1106, 1108, 1114 can be used to convert the lower member block 1010 to the lower member 122. For example, the templates can be aligned with the drilled blocks, a sharpie or another writing tool can be used to outline the edges of the templates, and the sharpie outline can be used as a guide for cutting, drilling, and sanding the drilled blocks to form the prosthetic knee components. The bottom block guide 1104 can be used to as a template for drilling the bore 133 that receives the pylon 110. The bottom block guide 1114 can be used to test the width and depth of the bore 133. The bottom block guide 1108 can be used as a template for cutting the inset in the lower member 122 that houses the lower end of the posterior link 126. The top block guide 1110 can be used as a template for cutting the profile of the upper member 120. The top block guide 1112 can be used as a template for cutting the inset in the upper member 120 that houses the upper end of the posterior link 126. The top block guide 1112 can also be used as a template for drilling a bore in the lower face of the upper member 120. The bore in the lower face of the upper member 122 can be used to insert the bolt 125 into the bore 134 in the upper face of the upper member 122, as shown in FIG. 1B. In some implementations, the drilled blocks 1010, 1012 are converted to finished upper and lower members according to other techniques, without use of the templates of FIG. 11.

In some implementations, a drilling jig includes additional drill sites for bores that are used for extension assist and/or locking mechanisms. Some locking mechanisms and some extension assist mechanisms require high precision bore placement. A drilling jig may include drill sites for such features. For example, a drilling jig may include drill sites for the bores that house the spring 550 and/or for the locking bores 542a, 542b shown in FIG. 5E, drill sites for the grooves 656 that receive the ends of the locking pin 650 and/or for the locking bores 642a, 642b in FIGS. 6A and 6B, and/or drill sites for other types of locking or extension assist mechanisms. In some implementations, extension assist and/or locking mechanisms can be implemented in a prosthetic knee without using the drilling jigs to drill any additional bores. For example, the bores needed for an extension assist or locking mechanism may require less precision than the bores for the mechanical axes of rotation and/or other bores.

In the examples shown in FIGS. 9A-9E and 10A-10D, a first drilling jig 900 is used to fabricate the links and a second drilling jig 1000 is used to fabricate the upper and lower members. A different number of drilling jigs may be used to fabricate a prosthetic knee. For example, an individual drilling jig may be used for each component of the knee *(i.e.,* a drilling jig for the posterior link, a drilling jig for the anterior links, a drilling jig for the upper member, and a drilling jig for the lower member). As another example, a single drilling jig may be used for all of the components 805. As such, other types of drilling jigs that have different configurations may be used in the manner described with respect to FIGS. 9A-9E and/or 10A-10D or in the same or a different manner to fabricate components of a prosthetic knee.

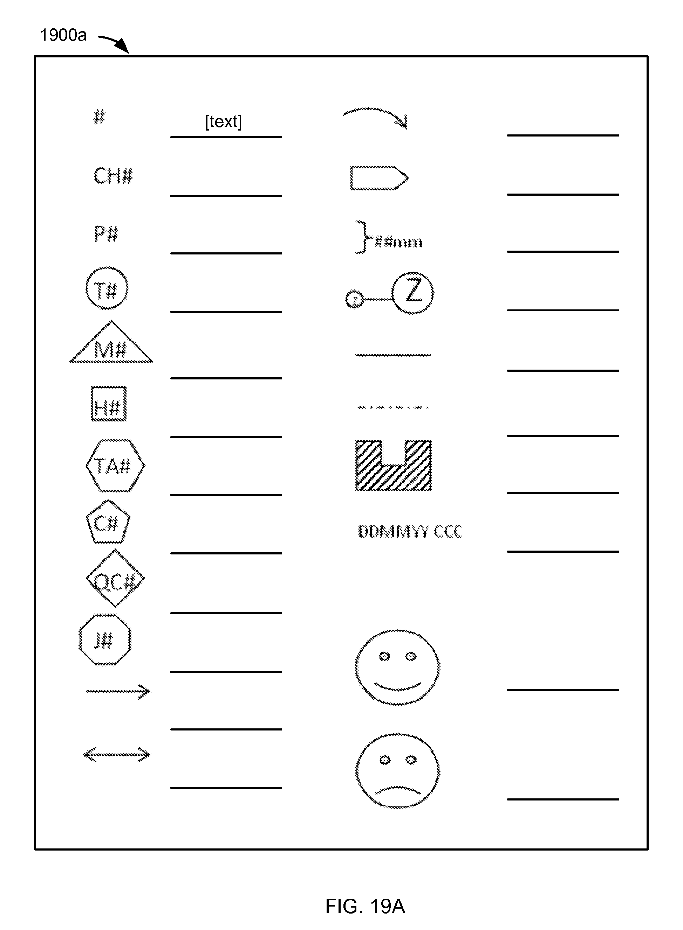

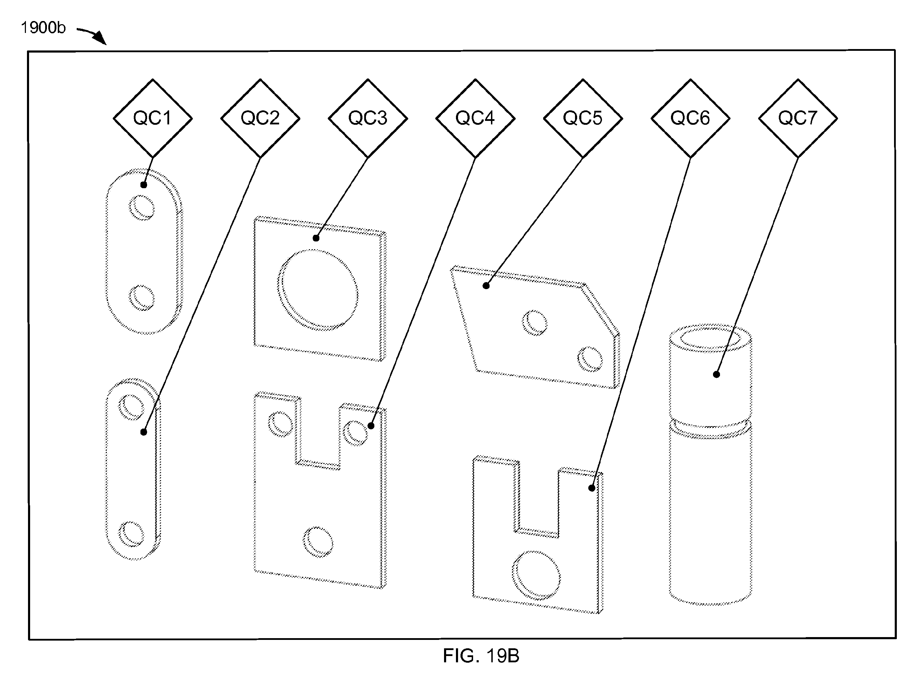

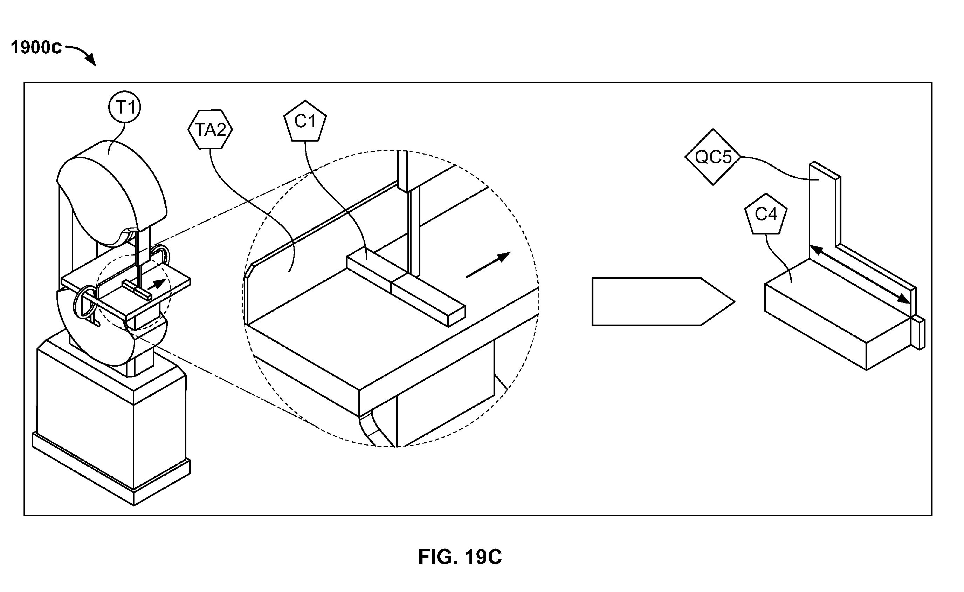

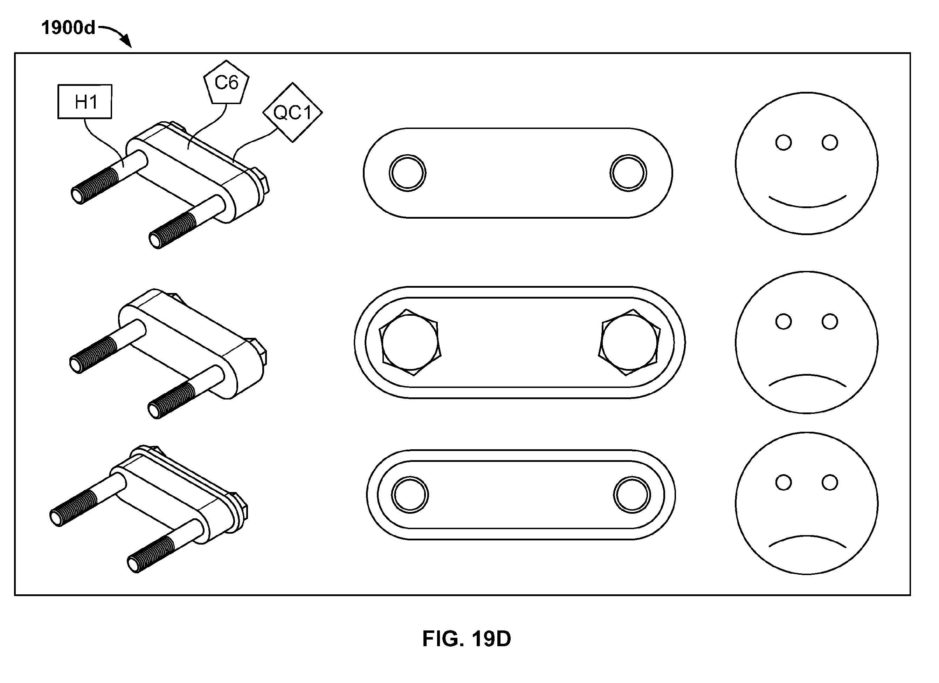

FIGS. 19A, 19B, 19C, and 19D are diagrams of an example graphical guide for manufacturing a polycentric prosthetic knee. FIG. 19A shows an example of a symbol index page 1900a from an example manufacturing guide. FIG. 19B shows an example of a parts page 1900b from an example manufacturing guide, showing example parts for a prosthetic knee manufacturing process. FIG. 19C shows an example cutting instruction page 1900c from an example manufacturing guide, showing instructions for an example cutting operation in a prosthetic knee manufacturing process. FIG. 19D shows an example quality control instruction page 1900d from an example manufacturing guide, showing instructions for implementing quality control in a prosthetic knee manufacturing process. A prosthetic knee manufacturing guide may include tens or hundreds of different or additional pages not shown in the figures. The example diagrams shown in FIGS. 19A-19D are selected to demonstrate features of a graphical manufacturing guide.

The graphical manufacturing guide allows polycentric prosthetic knees and/or component parts to be safely and reliably manufactured, assembled, deployed, maintained, and/or repaired by laborers having simple skill sets, using common, inexpensive tools and materials. For example, the step-by-step graphical instructions allow the prosthetic knee 108a shown in FIGS. 1B, 1C, 1D, 1F, and 1G and/or other embodiments shown and described herein to be manufactured without access to medical personnel or technicians having advanced training and/or without the need for advanced tools, materials, or manufacturing equipment. Such a manufacturing guide may be beneficial in clinics and for amputees across the developing world, for example, in locations and conditions where certain types of medical care, manufacturing facilities, and/or materials are not frequently accessible.

The graphical manufacturing guide may be written in a manner that allows the guide to be efficiently deployed in many different cultures across the developing world. For example, the graphical manufacturing guide may include a minimal amount of words or text that would have to be translated for different cultures, regions, languages, and dialects. As such, the graphical manufacturing guide may include one or a small number of pages of text that can be quickly and/or inexpensively translated for local deployment, and the remaining pages of the guide can be deployed without needing to be translated. As another example, the graphical manufacturing guide may include symbols that are recognized and understood by people of varying ethnic, linguistic, and cultural backgrounds. Such symbols may be culturally neutral, in that their meaning can be readily understood by people of multiple different cultures.

The symbol index page 1900a of FIG. 19A includes culturally neutral symbols that can be used in a graphical manufacturing guide. The blank line beside each symbol in the diagram 1900a represents a space where text may be inserted to indicate the meaning of the associated symbol. The text may be provided in any language, for example, English, Hindi, French, Spanish, Mandarin, Arabic, and/or others. When the graphical manufacturing guide is deployed, the text in the symbol index page 1900a can be translated, and the remaining pages of the guide may require minimal or no translation. The graphical manufacturing guide may allow a prosthetic knee to be manufactured locally, which may reduce the cost of the prosthetic knee and may allow for more convenient access to repair services and parts.

Several example alphanumeric symbols are shown in FIG. 19A. The "#" symbol can serve as a placeholder for any number or digit. "CH#" can be used to represent chapter numbers of the manual (*e.g.*, CH1 can represent chapter 1, CH2 can represent chapter 2, etc.). "P#" can be used to represent page numbers of the manual (*e.g.*, "P#1" can represent page 1, "P#2" can represent page 2, etc.), "T#" can be used to represent a tool number (e.g., T1 can represent a band saw, T2 can represent a drill press, etc.). The symbol T1 is used in the example cutting instruction page 1900c to indicate the band saw. "M#" can represent a raw material number (*e.g*., M1 can represent Delrin round stock, M2 can represent Delrin flat stock etc.). "H#" can represent a hardware part number (*e.g*, H1 can represent bolts, H2 can represent nuts, etc.). The symbol H1 is used in the quality control page 1900d to indicate the hex bolts. "TA#" can represent a tool accessory number (*e.g.*, TA1 can represent a drill bit, TA2 can represent a saw fence, etc.). The symbol TA2 is used in the example cutting instruction page 1900c to indicate the saw fence. "C#" can represent a component number for a component of the prosthetic knee (*e.g.,* C1 can represent the upper member, C2 can represent the lower member, etc.). The symbols C1, C4, and C6 are used in the example cutting instruction page 1900c and the example quality control page 1900d to indicate components of a prosthetic knee. "QC#" can represent a quality control part number (*e.g.*, QC1 can represent one of the templates, etc.). Several "QC#" symbols are used in the example parts page 1900b, the example cutting instruction page 1900c, and the example quality control page 1900d. "J#" can represent a drilling jig component number (*e.g.*, J1 can represent a drilling jig cover, etc.). The symbol "##mm" may be used to represent a specification length of a component part (*e.g.,* 25 mm length, 60 mm length, etc.). As shown in FIGS. 19A-19C, the geometric shape can be associated with each type of alphanumeric symbol for consistency and cross-lingual comprehension. For example, the "T#" symbol may be represented in a circle, the "M#" symbol may be represented in a triangle, etc. Different and/or additional geometric shapes may be associated with each type of alphanumeric symbol. One or more of the alphanumeric symbols may be presented without an associated geometric symbol.

Several example arrow symbols are shown in FIG. 19A. A first type of arrow (*e.g.*, arrow head at only one end) may indicate single direction movement in a straight line. A second type of arrow (*e.g.*, arrow heads at both ends) may indicate movement in two directions in a straight line. A third type of arrow (*e.g.*, curved line with arrow head) may indicate movement not in a straight line, such as rotation. A fourth type of arrow (*e.g.*, an open geometric arrow) may indicate a direction of progressing through steps displayed on a page.

Other example symbols are shown in FIG. 19A. For example, a zoom symbol may be used to indicate a close-up view of an illustrated object. A solid line may indicate where a line should be drawn (*e.g*., on a component). A dashed or chain line may indicate where a saw will cut but no line is drawn on the object. A cutaway symbol may indicate where the inside of an object is shown. A part stamping format DDMMYY CCC may indicate a convention for labeling manufactured parts. For example, DD may indicate the day, MM may indicate the month, YY may indicate the year, and CCC may indicate the clinic or location where the component was manufactured. As a particular example, a part manufactured on 19 May 2010 in clinic number 125 would be stamped "190510 125". A smiling face symbol may represent "ok," "yes," and "good." A frowning face symbol may represent "not ok," "no," and "bad."

FIG. 12A is a plan view of a cover component 1202 of an example drilling jig. FIG. 12B is a side view of the cover component 1202 of FIG. 12A. The cover component 1202 may be used with a slot component to form a drilling jig that is used for drilling link components of a prosthetic knee. For example, the cover component 1202 can be the cover component 914 of FIGS. 9A-9D.

The example drilling jig cover component 1202 includes six bolt holes 1208 for securing the cover component 1202 to a slot component, such as the slot component 1302 of FIGS. 13A-13C. The example drilling jig cover component 1202 includes six cavities 1206 that allow two bores to be drilled through the full width of the link block residing in the slot component. The cavities 1206 may each align with a corresponding drilling site on a slot component of the drilling jig.

FIG. 13A and 13C are plan views of a slot component 1302 of an example drilling jig. FIG. 13B is a cross-sectional view of the slot component 1302 of FIG. 13A. The slot component 1302 may be used with a cover component to form a drilling jig that is used for drilling link components of a prosthetic knee. For example, the slot component 1302 can be the slot component 902 of FIGS. 9A-9D.

The slot component 1302 includes one posterior link slot 1308 that can receive a posterior link block, such as the posterior link block 910 of FIG. 9A. The slot component 1302 includes two anterior link slots 1310 that can each receive an anterior link block, such as the anterior link blocks 912a, 912b of FIG. 9A. Each of the slots 1308, 1310 includes a reference corner 1316. Pressure bolts may be applied to the link blocks in the slots 1308, 1310 to force the link block to the reference corner 1316 of the slot in which it resides. Forcing the link blocks to the reference corner may improve consistency, precision, and interchangeability of prosthetic knee components fabricated using the drilling jig.

The example drilling jig slot component 1302 includes six bolt holes 1314 for securing a cover component, such as the cover component 1202 of FIGS. 12A and 12B, to the slot component 1302. The example drilling jig slot component 1302 includes six drill sites 1306 that allow two bores to be drilled through the full width of the three link blocks residing in the slots 1308, 1310. FIG. 13C shows some example dimensions for the locations of drilling sites 1306 in the example slot component. The dimensions shown are in units of millimeters (mm). Each of the drilling sites 1306 may align with a corresponding cavity in a cover component of the drilling jig.

FIG. 14 are plan views of a cover component 1402 of an example drilling jig. The cover component 1402 may be used with a slot component to form a drilling jig that is used for drilling upper member and lower member components of a prosthetic knee. For example, the cover component 1402 can be the cover component 1014 of FIGS. 10A-10C.

The example drilling jig cover component 1402 includes six bolt holes 1406 for securing the cover component 1402 to a slot component, such as the slot component 1502 of FIGS. 15A-15H. The example drilling jig cover component 1402 includes five drill sites 1404 that allow bores to be drilled through the full width of the blocks residing in the slot component. The drill sites 1404 may each align with a corresponding cavity on a slot component of the drilling jig. The example drilling jig cover component 1402 also includes bolt holes 1408 for pressure bolts that secure the blocks in a reference corner in the slot component.

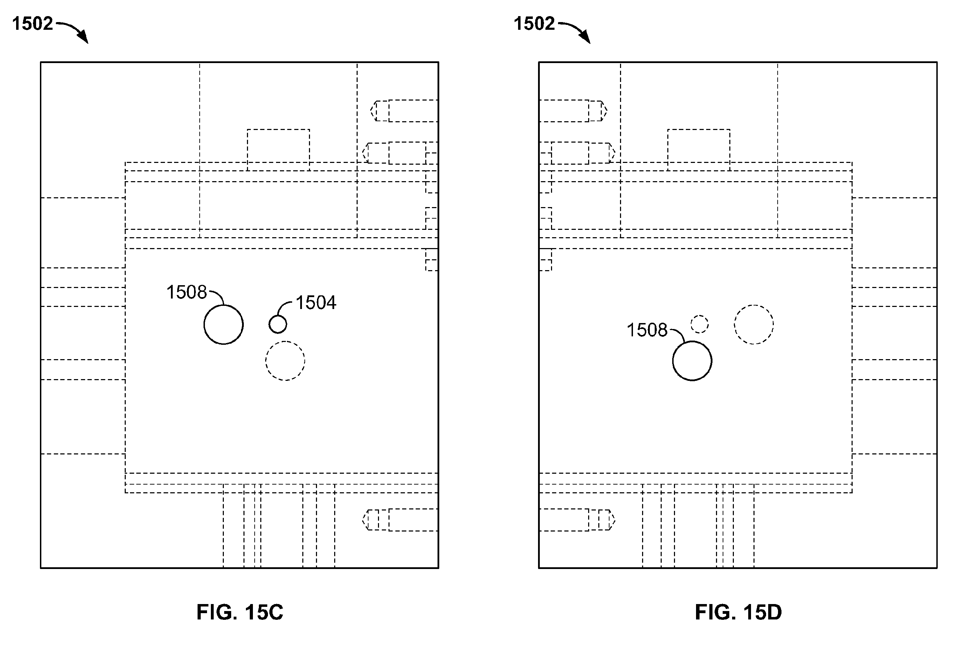

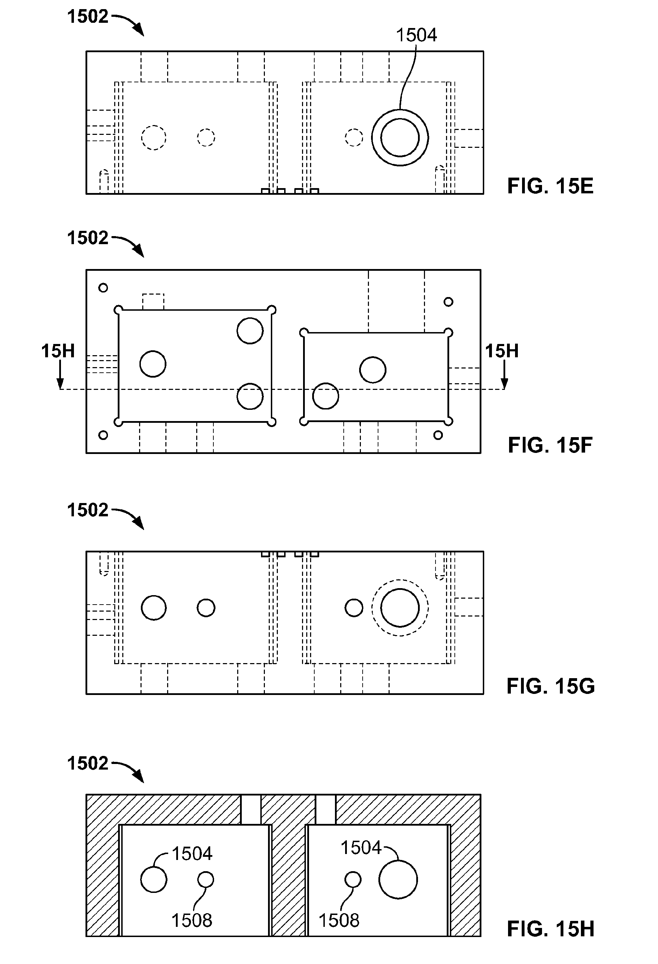

FIGS. 15A, 15B, and 15F are plan views of a slot component 1502 of an example drilling jig. FIG. 15C is a side view of a first side of the slot component 1502 of FIG. 15A. FIG. 15D is a side view of a second side of the slot component 1502 of FIG. 15A. FIG. 15E is a side view of a third side of the slot component 1502 of FIG. 15A. FIG. 15G is a side view of a fourth side of the slot component 1502 of FIG. 15A. FIG. 15H is a cross-sectional view of the slot component 1502 of FIG. 15A. The slot component 1502 may be used with a cover component to form a drilling jig that is used for drilling upper member and lower member components of a prosthetic knee. For example, the slot component 1502 can be the slot component 1002 of FIGS. 10A and 10B.

The slot component 1502 includes a lower member slot 1510 that can receive a lower member block, such as the lower member block 1010 of FIG. 10A. The slot component 1502 includes an upper member slot 1512 that can receive an upper member block, such as the upper member block 1012 of FIG. 10A. Each of the slots 1510, 1512 includes a reference corner 1516. Pressure bolts may be applied to the upper and lower blocks in the slots 1510, 1512 to force the blocks to the reference corner 1516 of the slot in which it resides. Pressure bolts may be applied through the pressure bolt holes 1508 in the slot component 1502 and the bolt holes 1408 in the cover component 1402. Forcing the blocks to the reference corner may improve consistency, precision, and interchangeability of prosthetic knee components fabricated using the drilling jig.

The example drilling jig slot component 1502 includes six bolt holes 1506 for securing a cover component, such as the cover component 1402 of FIG. 14, to the slot component 1502. The example drilling jig slot component 1502 includes several drill sites 1514 shown in FIGS. 15A-15H that allow bores to be drilled through blocks residing in the slots 1510, 1512. FIG. 15B shows some example dimensions for the locations of some of the drilling sites 1514 in the example slot component. The dimensions shown are in units of millimeters (mm). Each of the drill sites 1514 may align with a corresponding drill site or cavity in a cover component of the drilling jig. One or more of the drill sites may be implemented as a cavity, for example, when there is an opposing drill site.

Similarly, while operations are depicted in the drawings in a particular order, this should not be understood as requiring that such operations be performed in the particular order shown or in sequential order, or that all illustrated operations be performed, to achieve desirable results.

Thus, particular implementations of the subject matter have been described. Nevertheless, various modifications may be made. Accordingly, other implementations are within the scope of the following claims.

## Claims

1. A polycentric prosthetic knee (108) comprising:
a plastic upper member (120) carrying a first upper axis (130a) and a second upper axis (128a);
a plastic lower member (122) carrying a first lower axis (130b) and a second lower axis (128b);
a first plastic link (124a) linking the first upper axis (130a) to the first lower axis (130b), the first plastic link (124a) rotatable with respect to the plastic upper member (120) about the first upper axis (130a) and rotatable with respect to the plastic lower member (122) about the first lower axis (130b); a second plastic link (126) linking the second upper axis (128a) to the second lower axis, the second plastic link rotatable with respect to the plastic upper member (120) about the second upper axis (128a) and rotatable with respect to the plastic lower member (122) about the second lower axis (128b); and a third plastic link (124b) linking the first upper axis (130a) to the first lower axis (130b), the third plastic link (124b) rotatable with respect to the plastic upper member (120) about the first upper axis (130a) and rotatable with respect to the plastic lower member (122) about the first lower axis (130b), the plastic lower member (122) rotatable with respect to the plastic upper member (120) about an instantaneous center of rotation (131) defined by the first upper axis (130a), the second upper axis (128a), the first lower axis (130b), and the second lower axis (128b).

2. The polycentric prosthetic knee (108) of claim 1, wherein the first plastic link (124a) comprises a first anterior link, the second plastic link (126) comprises a posterior link, and the third plastic link (124b) comprises a second anterior link.

3. The polycentric prosthetic knee (108) of any of the preceding claims, further comprising an extension assist mechanism that biases the prosthetic knee toward an extended position.

4. The polycentric prosthetic knee (108) of claim 3, wherein the extension assist mechanism comprises one of:
a rubber band (138, 172) that biases the prosthetic knee toward an extended position; or
a coil spring (550) having a first end secured to the plastic upper member (120) and a second end secured to the first plastic link (124a), the coil spring (550) biases the prosthetic knee (108) toward an extended position.

5. The polycentric prosthetic knee (108, 1600) of any of the preceding claims, further comprising a locking mechanism operable to prevent rotation of the plastic lower member (1622) with respect to the plastic upper member (1620).

6. The polycentric prosthetic knee (108, 1600) of claim 5, wherein the locking mechanism comprises a locking pin (1640).

7. The polycentric prosthetic knee (108) of any of the preceding claims, further comprising:
a pylon bore in the plastic lower member (122) adapted to receive an end of a pylon; and
a plurality of fastener bores in the plastic lower member (122) substantially perpendicular to the pylon bore and extending from an outer surface of the plastic lower member (122) to the pylon bore, two of the fastener bores parallel to the first lower axis (130b) and the second lower axis (128b), two of the fastener bores perpendicular to the first lower axis (130b) and the second lower axis (128b).

8. The polycentric prosthetic knee (108) of any of the preceding claims, wherein at least one of the plastic upper member (120), the plastic lower member (122), the first plastic link (124a), the second plastic link (126), or the third plastic link (124b) comprises a load-bearing plastic structure made of homopolymer polyacetal plastic.

9. The polycentric prosthetic knee (108) of any of the preceding claims, wherein at least one of the plastic upper member (120), the plastic lower member (122), the first plastic link (124a), the second plastic link (126), or the third plastic link (124b) comprises a load-bearing plastic structure made of copolymer polyacetal plastic.

10. The polycentric prosthetic knee of any of the preceding claims, wherein the polycentric prosthetic knee can support an external static load of 4,480 Newtons.

11. A method of manufacturing a polycentric prosthetic knee of any of the preceding claims, the method comprising:
fabricating plastic components (805) for the polycentric prosthetic knee from plastic source material (801), the plastic components comprising the plastic upper member (120), the plastic lower member (122), the first plastic link (124a), the second plastic link (126), and the third plastic link (124b);
assembling the plastic components to build the polycentric prosthetic knee (108) comprising:
the plastic upper member (120) carrying the first upper axis (130a) and the second upper axis (128a);
the plastic lower member (122) carrying the first lower axis (130b) and the second lower axis (128b), the first plastic link (124a) linking the first upper axis (130a) to the first lower axis (130b), the second plastic link (126) linking the second upper axis (128a) to the second lower axis (128b), the third plastic link (124b) linking the first upper axis (130a) to the first lower axis (130b), the plastic lower member (122) rotatable with respect to the plastic upper member (120) about the instantaneous center of rotation (131) defined by the first upper axis (130a), the second upper axis (128a), the first lower axis (130b), and the second lower axis (128b).

12. The method claim 11, wherein the plastic source material comprises at least one of homopolymer polyacetal plastic or comopolymer polyacetal plastic.

13. The method of any of claims 11 through 12, wherein fabricating the plastic components comprises:
providing a plurality of plastic blocks in a drilling jig (900), the plastic blocks formed from the plastic source material; and
using the drilling jig (900) to guide a drill bit that forms a plurality of bores in each of the plurality of plastic blocks.

14. The method of claim 13, wherein the drilling jig (900) comprises:
a housing defining an inner cavity to receive a first plastic block;
a plurality of pressure members extending into the inner cavity and adapted to force the first plastic block toward a reference location in the inner cavity; and
a plurality of drill-site (922) bores extending from an outer surface of the housing to the inner cavity, the plurality of drill-site bores adapted to guide the drill bit into the first inner cavity to drill the plurality of bores through the first block, the plurality of bores comprising a first bore adapted to carry a first rotational axis of the polycentric prosthetic knee and a second bore adapted to carry a second rotational axis of the polycentric prosthetic knee.

15. The method of claim 14, the plurality of drill-sites (922) adapted to guide the drill bit to form the plurality bores within a specified distance from each other, the plurality of drill-sites adapted to guide the drill bit to form the first plurality bores within a specified runout tolerance.

## Patentansprüche

1. Eine polyzentrische Knieprothese (108) umfassend:
ein oberes Kunstoffelement (120) eine erste obere Achse (130a) und eine zweite obere Achse (128a) tragend;
eine unteres Kunststoffelement (122) eine erste untere Achse (130b) und eine zweite untere Achse (128b) tragend;
eine erste Kunsttoffverbindung (124a) die die erste obere Achse (130a) mit der ersten unteren Achse (130b) verbindet, wobei die erste Kunsttoffverbindung (124a) drehbar ist bezüglich des oberes Kunstoffelements (120) um die erste obere Achse (130a), und drehbar ist bezüglich des unteren Kunstoffelements(122) um die erste untere Achse (130b);
eine zweite Kunsttoffverbindung (126) die die zweite obere Achse (128a) mit der zweiten unteren Achse verbindet, wobei die zweite Kunsttoffverbindung drehbar ist bezüglich des oberes Kunstoffelements (120) um die zweite obere Achse (128a), und drehbar ist bezüglich des unteren Kunstoffelements (122) um die zweite untere Achse (128b); und
eine dritte Kunsttoffverbindung (124b) die die erste obere Achse (130a) mit der ersten unteren Achse (130b) verbindet, wobei die erste Kunsttoffverbindung (124b) drehbar ist bezüglich des oberes Kunstoffelements (120) um die erste obere Achse (130a), und drehbar ist bezüglich des unteren Kunstoffelements (122) um die erste untere Achse (130b),
wobei das unteren Kunstoffelement (122) drehbar ist bezüglich des oberen Kunstoffelements (120) um einen unmittelbaren Rotationspunkt (131) welcher von der ersten oberen Achse (130a), der zweiten oberen Achse (128a), der ersten untern Achse (130b) und der zweiten unteren Achse (128b) definiert wird.

2. Die polyzentrische Knieprothese (108) nach Anspruch 1, wobei die erste Kunsttoffverbindung (124a) eine erste vordere Verbindung umfasst, und wobei die zweite Kunsttoffverbindung (126) eine hintere Verbindung umfasst, und wobei die dritte Kunsttoffverbindung (124b) eine zweite vordere Verbindung umfasst.

3. Die polyzentrische Knieprothese (108) nach irgendeinem der vorhergegangenen Ansprüche, umfasst des Weiteren einen Mechanismus zur Unterstützung des Streckens der die Knieprothese in Richtung einer gestreckten Position vorspannt.

4. Die polyzentrische Knieprothese (108) nach Anspruch 3, wobei der Mechanismus zur Unterstützung des Streckens eines von folgenden umfasst:
ein Gummiband (138, 172), das die Knieprothese in Richtung einer gestreckten Position ausrichtet; oder
eine Spiralfeder (550) dessen erstes Ende an dem oberen Kunststoffelement (120) befestigt ist, und ein zweites Ende an der ersten Kunststoffverbindung (124a) befestigt ist, wobei die Spiralfeder (550) die Knieprothese in Richtung einer gestreckten Position ausrichtet.

5. Die polyzentrische Knieprothese (108, 1600) nach irgendeinem der vorhergegangenen Ansprüche, umfasst des Weiteren einen Verriegelungsmechanismus bedienbar um die Drehung des unteren Kunststoffelements (1622) bezüglich des oberen Kunststoffelements ( 1620) zu unterbinden.

6. Die polyzentrische Knieprothese (108, 1600) nach Anspruch 5, wobei der Verriegelungsmechanismus einen Verriegelungsstift (1640) umfasst.

7. Die polyzentrische Knieprothese (108) nach irgendeinem der vorhergegangenen Ansprüche, umfasst des Weiteren:
eine Pylonbohrung in dem unteren Kunststoffelement (122) geeignet um ein Ende eines Pylons aufzunehmen; und
und eine Mehrzahl von Befestigungsbohrungen in dem unteren Kunststoffelement (122) die im wesentlichen senkrecht zu der Pylonbohrung sind und sich von einer äußeren Oberfläche des unteren Kunststoffelement (122) zu der Pylonbohrung erstrecken, wobei zwei der Befestigungsbohrungen parallel zu der ersten unteren Achse (130b) und
der zweiten unteren Achse (128b) sind, und wobei zwei der Befestigungsbohrungen senkrecht zu der der ersten unteren Achse (130b) und der zweiten unteren Achse (128b) sind.

8. Die polyzentrische Knieprothese (108) nach irgendeinem der vorhergegangenen Ansprüche, wobei mindestens eines von dem oberen Kunststoffelement (120), dem unteren Kunststoffelement (122), der erste Kunsttoffverbindung (124a), der zweite Kunsttoffverbindung (126) oder der dritte Kunsttoffverbindung (124b), eine tragende Kunststoffstruktur umfasst, die aus Homopolymer Polyacetal Kunststoff hergestellt ist.

9. Die polyzentrische Knieprothese (108) nach irgendeinem der vorhergegangenen Ansprüche, wobei mindestens eines von dem oberen Kunststoffelement (120), dem unteren Kunststoffelement (122), der erste Kunsttoffverbindung (124a), der zweite Kunsttoffverbindung (126) oder der dritte Kunsttoffverbindung (124b), eine tragende Kunststoffstruktur umfasst, die
aus Copolymer Polyacetal Kunststoff hergestellt ist.

10. Die polyzentrische Knieprothese (108) nach irgendeinem der vorhergegangenen Ansprüche, wobei die polyzentrische Knieprothese eine externen statischen Belastung von 4,480 Newton aushält.

11. Ein Verfahren zur Herstellung einer polyzentrischen Knieprothese nach irgendeinem der vorhergegangenen Ansprüche, wobei das Verfahren umfasst:
herstellen von Kunststoffkomponenten (805) für die polyzentrische Knieprothese aus Kunststoffausgangsmaterial (801), wobei die Kunststoffkomponenten umfassen das obere Kunstoffelement (120), das untere Kunststoffelement (122), die erste Kunsttoffverbindung (124a) die zweite Kunsttoffverbindung (126), und die dritte Kunsttoffverbindung (124b);
montieren der Kunststoffkomponenten um die polyzentrischen Knieprothese (108) zu bilden umfassend:
das obere Kunstoffelement (120) die erste obere Achse (130a) und diezweite obere Achse (128a) tragend;
das untere Kunststoffelement (122) die erste untere Achse (130b) und diezweite untere Achse (128b) tragend;
die erste Kunsttoffverbindung (124a) die die erste obere Achse (130a) mit der ersten unteren Achse (130b) verbindet,
die zweite Kunsttoffverbindung (126) die die zweite obere Achse (128a) mit der zweiten unteren Achse verbindet, und
die dritte Kunsttoffverbindung (124b) die die erste obere Achse (130a) mit der ersten unteren Achse (130b) verbindet, wobei das unteren Kunstoffelement (122) drehbar ist bezüglich des oberes Kunstoffelements (120) um den unmittelbaren Rotationspunkt (131) welcher von der ersten oberen Achse (130a), der zweiten oberen Achse (128a), der ersten untern Achse (130b) und der zweiten unteren Achse (128b) definiert wird.

12. Das Verfahren nach Anspruch 11, wobei das Kunststoffausgangsmaterial mindestens eines von Homopolymer Polyacetal Kunststoff oder Copolymer Polyacetal Kunststoff umfasst.

13. Das Verfahren nach irgendeinem der Ansprüche 11 bis 12, wobei Herstellen von Kunststoffkomponenten umfassend:
Bereitstellen einer Mehrzahl von Kunststoffblöcken in eine Bohrvorrichtung (900), wobei die Kunststoffblöcke aus dem Kunststoffausgangsmaterial geformt sind; und
Verwenden der Bohrvorrichtung (900) um eine Bohrspitze zu führen die eine Mehrzahl von Bohrungen in jedes der Mehrzahl der Kunststoffblöcke formt.

14. Das Verfahren nach Anspruch 13, wobei die Bohrvorrichtung (900) umfasst:
ein Gehäuse, das eine innere Kavität definiert um einen ersten Kunststoffblock aufzunehmen;
eine Mehrzahl von Presselementen die sich in die innere Kavität erstrecken und ausgelegt sind um den Kunststoffblock in Richtung einer Referenzposition in der inneren Kavität zu pressen; und eine Mehrzahl von Bohrstellen (922) Bohrungen die sich von einer äußeren Oberfläche des Gehäuses in die innere Kavität erstrecken, wobei die Mehrzahl von Bohrstellen (922) Bohrungen ausgelegt ist um die Bohrspitze in die erste innere Kavität zu führen um die Mehrzahl von Bohrungen durch den ersten Block zu bohren, wobei die Mehrzahl von Bohrungen umfasst eine erste Bohrung die ausgelegt ist um eine erste Rotationsachse der polyzentrischen Knieprothese zu tragen, und eine zweite Bohrung die ausgelegt ist um eine zweite Rotationsachse der polyzentrischen Knieprothese zu tragen.

15. Das Verfahren nach Anspruch 14, wobei die Mehrzahl von Bohrstellen (922) ausgelegt ist um die Bohrspitze zu führen um die Mehrzahl von Bohrungen innerhalb einer festgelegten Entfernung von einander zu bilden, wobei die Mehrzahl von Bohrstellen ausgelegt ist um die Bohrspitze zu führen um die Mehrzahl von Bohrungen innerhalb einer festgelegten Rundlauftoleranz zu bilden.

## Revendications

1. Genou (108) prothétique polycentrique comportant :
un élément (120) supérieur en plastique portant un premier axe (130a) supérieur et un deuxième axe (128a) supérieur ;
un élément (122) inférieur en plastique portant un premier axe (130b) inférieur et un deuxième axe (128b) inférieur ;
un premier lien (124a) en plastique reliant le premier axe (130a) supérieur au premier axe (130b) inférieur, le premier lien (124a) en plastique pouvant être entraîné en rotation par rapport à l'élément (120) supérieur en plastique autour du premier axe (130a) supérieur et pouvant être entraîné en rotation par rapport à l'élément (122) inférieur en plastique autour du premier axe (130b) inférieur ; un deuxième lien (126) en plastique reliant le deuxième axe (128a) supérieur au deuxième axe inférieur, le deuxième lien en plastique pouvant être entraîné en rotation par rapport à l'élément (120) supérieur en plastique autour du deuxième axe (128a) supérieur et pouvant être entraîné en rotation par rapport à l'élément (122) inférieur en plastique autour du deuxième axe (128b) inférieur ; et un troisième lien (124b) en plastique reliant le premier axe (130a) supérieur au premier axe (130b) inférieur, le troisième lien (124b) en plastique pouvant être entraîné en rotation par rapport à l'élément (120) supérieur en plastique autour du premier axe (130a) supérieur et pouvant être entraîné en rotation par rapport à l'élément (122) en plastique inférieur autour du premier axe (130b) inférieur, l'élément (122) inférieur en plastique pouvant être entraîné en rotation par rapport à l'élément (120) supérieur en plastique autour d'un centre instantané de rotation (131) délimité par le premier axe (130a) supérieur, le deuxième axe (128a) supérieur, le premier axe (130b) inférieur, et le deuxième axe (128b) inférieur.

2. Genou (108) prothétique polycentrique selon la revendication 1, le premier lien (124a) en plastique comportant un premier lien antérieur, le deuxième lien (126) en plastique comportant un lien postérieur, le troisième lien (124b) en plastique comportant un deuxième lien antérieur.

3. Genou (108) prothétique polycentrique selon l'une quelconque des revendications précédentes, comportant en outre un mécanisme d'aide à l'extension qui sollicite le genou prothétique vers une position étendue.

4. Genou (108) prothétique polycentrique selon la revendication 3, le mécanisme d'aide à l'extension comportant :
une bande (138, 172) élastique qui sollicite le genou prothétique vers une position étendue ; ou
un ressort (550) hélicoïdal qui présente une première extrémité fixée à l'élément (120) supérieur en plastique et une deuxième extrémité fixée au premier lien (124a) en plastique, le ressort (550) hélicoïdal sollicitant le genou (108) prothétique vers une position étendue.

5. Genou (108, 1600) prothétique polycentrique selon l'une quelconque des revendications précédentes, comportant en outre un mécanisme de verrouillage servant à empêcher la rotation de l'élément (1622) inférieur en plastique par rapport à l'élément (1620) supérieur en plastique.

6. Genou (108, 1600) prothétique polycentrique selon la revendication 5, le mécanisme de verrouillage comportant une goupille (1640) de verrouillage.

7. Genou (108) prothétique polycentrique selon l'une quelconque des revendications précédentes, comportant en outre :
un trou pour pilon dans l'élément (122) inférieur en plastique conçu pour recevoir une extrémité d'un pilon ; et
une pluralité de trous pour dispositifs de fixation dans l'élément (122) inférieur en plastique sensiblement perpendiculaires au trou de pilon et s'étendant depuis une surface externe de l'élément (122) inférieur en plastique au trou de pilon, deux des trous pour dispositifs de fixation étant parallèles au premier axe (130b) inférieur et au deuxième axe (128b) inférieur, deux des trous pour dispositifs de fixation étant perpendiculaires au premier axe (130b) inférieur et au deuxième axe (128b) inférieur.

8. Genou (108) prothétique polycentrique selon l'une quelconque des revendications précédentes, au moins l'élément (120) supérieur en plastique, l'élément (122) inférieur en plastique, le premier lien (124a) en plastique, le deuxième lien (126) en plastique, et/ou le troisième lien (124b) en plastique comportant une structure en plastique porteuse de charge constituée de plastique polyacétalique homopolymère.

9. Genou (108) prothétique polycentrique selon l'une quelconque des revendications précédentes, au moins l'élément (120) supérieur en plastique, l'élément (122) inférieur en plastique, le premier lien (124a) en plastique, le deuxième lien (126) en plastique, et/ou le troisième lien (124b) en plastique comportant une structure en plastique porteuse de charge constituée de plastique polyacétalique copolymère.

10. Genou prothétique polycentrique selon l'une quelconque des revendications précédentes, le genou prothétique polycentrique pouvant supporter une charge statique extérieure de 4480 newtons.

11. Procédé de fabrication d'un genou prothétique polycentrique selon l'une quelconque des revendications précédentes, le procédé consistant à :
fabriquer des composants (805) en plastique pour le genou prothétique polycentrique dans un matériau (801) de source en plastique, les composants en plastique comportant l'élément (120) supérieur en plastique, l'élément (122) inférieur en plastique, le premier lien (124a) en plastique, le deuxième lien (126) en plastique, et le troisième lien (124b) en plastique ;
assembler les composants en plastique pour construire le genou (108) prothétique polycentrique comportant :
l'élément (120) supérieur en plastique portant le premier axe (130a) supérieur et le deuxième axe (128a) supérieur ;
l'élément (122) inférieur en plastique portant le premier axe (130b) inférieur et le deuxième axe (128b) inférieur, le premier lien (124a) en plastique reliant le premier axe (130a) supérieur au premier axe (130b) inférieur, le deuxième lien (126) en plastique reliant le deuxième axe (128a) supérieur au deuxième axe (128b) inférieur, le troisième lien (124b) en plastique reliant le premier axe (130a) supérieur au premier axe (130b) inférieur, l'élément (122) inférieur en plastique pouvant être entraîné en rotation par rapport à l'élément (120) supérieur en plastique autour du centre instantané de rotation (131) délimité par le premier axe (130a) supérieur, le deuxième axe (128a) supérieur, le premier axe (130b) inférieur, et le deuxième axe (128b) inférieur.

12. Procédé selon la revendication 11, le matériau de source en plastique comportant au moins du plastique polyacétalique homopolymère et/ou du plastique polyacétalique copolymère.

13. Procédé selon l'une quelconque des revendications 11 à 12, la fabrication des composants en plastique consistant à :
fournir une pluralité de blocs en plastique dans un gabarit (900) de perçage, les blocs en plastique étant formés dans le matériau de source en plastique ; et
utiliser le gabarit de perçage (900) pour guider une mèche de perceuse qui forme une pluralité de trous dans chaque bloc de la pluralité de blocs en plastique.

14. Procédé selon la revendication 13, le gabarit (900) de perçage comportant :
un boîtier délimitant une cavité interne destinée à recevoir un premier bloc en plastique ;
une pluralité d'éléments de pression s'étendant dans la cavité interne et conçus pour forcer le premier bloc en plastique vers un emplacement de référence dans la cavité interne ; et
une pluralité de trous correspondant aux sites de perçage (922) s'étendant d'une surface externe du boîtier à la cavité interne, la pluralité de trous correspondant aux sites de perçage étant conçue pour guider le gabarit de perçage dans la première cavité interne afin de percer la pluralité de trous à travers le premier bloc, la pluralité de trous comportant un premier trou conçu pour porter un premier axe de rotation du genou prothétique polycentrique et un deuxième trou conçu pour porter un deuxième axe de rotation du genou prothétique polycentrique.

15. Procédé selon la revendication 14, la pluralité de sites de perçage (922) étant conçue pour guider la mèche de perceuse afin de former la pluralité de trous à une distance spécifiée les uns des autres, la pluralité de sites de perçage étant conçue pour guider la mèche de perceuse afin de former la première pluralité de trous avec une tolérance de battement spécifiée.
